# EUROPEAN PATENT SPECIFICATION

(11) **EP 3 964 136 B1**
(45) Date of publication and mention of the grant of the patent: **14.05.2025**
(21) Application number: 20305972.0
(22) Date of filing: 02.09.2020
(51) Int. Cl.: A61B 8/08, A61B 8/00

(54) **SYSTEM AND METHOD FOR GUIDING A USER IN ULTRASOUND ASSESSMENT OF A FETAL ORGAN**
SYSTEM UND VERFAHREN ZUM FÜHREN EINES BENUTZERS BEI DER ULTRASCHALLBEURTEILUNG EINES FÖTALEN ORGANS
SYSTÈME ET PROCÉDÉ POUR GUIDER UN UTILISATEUR DANS L'ÉVALUATION PAR ULTRASONS D'UN ORGANE F TAL

(43) Date of publication of application: 09.03.2022
(73) Proprietor: Diagnoly, 69008 Lyon (FR)
(72) Inventor: VOZNYUK, Ivan, 69003 Lyon (FR); QUARELLO, Edwin, 13008 Marseille (FR)
(74) Representative: Icosa

(56) References cited:
- WO-A1-2019/201726
- US-A1- 2005 251 013

## Description

### FIELD OF INVENTION

The present invention pertains to the field of medical imaging. In particular, the invention relates to method and system for guiding a user in ultrasound assessment of a fetal organ so as to assess the quality of an examination and to detect pathological conditions.

### BACKGROUND OF INVENTION

A fetal abnormality is a defect in the fetus of a genetic and/or structural nature that can cause significant fetal health and developmental complications, maternal complicated delivery and lead to complex postnatal medical interventions and adverse outcomes [Mai2019].

According to the recent research work in the field of fetal development, congenital malformations occur in 3-5% of live births [Cdcp2008, Bower2010, Debost2014, TBDR2016]. They can range from minor issues that can be easily fixed, to severe ones meaning that the fetus will either be stillborn, die shortly after birth or be associated with a severe morbidity. Latter ones are responsible for the majority of lifelong disabilities and represent the leading cause of infant deaths [Decoufle2001, CDCP2015].

Detection of fetal anomalies allows to anticipate a tailored inborn birth, prompt medical care or surgical treatment in specialized centers at birth or in the neonatal period, as well as to provide an adequate information to the parents. For all of these reasons, the ability to detect fetal complications prenatally is critical.

Cardiac abnormalities are the most common and one of the most impacting group of malformations affecting both fetuses and newborn infants. Accounting for approximately 1% of live births [Sun2018], of which about 25% to 40% are severe forms [Jouannic2010, Sun2018], they are responsible for a half of the infant mortality due to malformations [Jouannic2010].

Improving the outcome of fetuses complicated by disease or abnormality(ies), such as cardiac for example, is the goal of fetal medicine. Detecting cardiac abnormalities using specific tools, implementing sophisticated techniques and screening strategies to diagnose foetal diseases, developing therapeutic tools, planning the tailored management of a fetus with a pathology: all of these actions constitute the subject of fetal medicine and aim to reduce the risks of disability of the child [Clur2012, Bensemlali2017].

For all these reasons, the medical community and society are making great efforts to screen for these prenatal conditions. Their prenatal detection improves the prognosis for the unborn child and the quality of life of the children concerned, who will be able to benefit from treatment [Berkley2009, Cha2012].

However, despite the progress in equipment, up to 52.8% of prenatal cardiac pathologies are unfortunately not detected today [Pinto2013]. This high rate is due to the fact that being highly "operator-dependent" as well as a "patient-dependent" examination, obstetric ultrasound remains one of the most complex and time-consuming techniques [Pinto2013, Sklansky2016]. As a result, these factors can generate errors in prenatal diagnosis and an overload of work involving a lack of time for health care personnel.

According to specialists [Bensemlali2016, vanNisselrooij2020], fetal heart disease is not always screened antenatally for 3 main reasons: (1) in 49% of cases, lack of skill in adapting ultrasound images (the ultrasound images obtained by the operator are not sufficient to make the correct diagnosis); (2) in 31% of the cases, lack of experience in diagnostics (the images obtained are good and the prenatal pathology is visible but not recognized by the operator); and finally (3), in 20% of cases, pathologies cannot be detected because they are not visible on the ultrasound images.

US2005251013A1 discloses a device for and method of automatic ultrasound view identification.

The present invention aims to overcome the above presented problems by proposing a system and a method configured to help the users to assess fetal ultrasound images and analyses these images in order to detect a large spectrum of abnormalities in fetal development.

### SUMMARY

The present invention relates to a computer-implemented method for guiding a user in ultrasound assessment of a fetal organ so as to assess the quality of an examination, based on an ultrasound images sequence comprising multiples predefined required views of the fetal organ, said method comprising:
- receiving in real time a sequence of 2D ultrasound images acquired by the user, each image comprising at least a portion of the fetal organ;
- providing each image as input to an image analysis structure DL1 comprising at least one first classifier DL1a, said first classifier DL1a being configured to identify if the image belongs to any view's category comprised in a predefined list of view's categories and, if so, to identify the view's category to which belongs the image among said predefined list of view's categories; wherein each view's category is associated to at least one predefined fetal anatomical landmark;
- providing each image as input to a second classifier DL1b of the image analysis structure, said second classifier DL1b being configured to detect the presence in the image of predefined fetal anatomical landmarks;
- whenever the first classifier DL1a identifies that the image corresponds to one view's category of the predefined list of view's categories and the second classifier DL1b identifies that a predefined number of fetal anatomical landmarks associated to the identified view's category are present in the image, adding said image to a valid images list;
- providing the valid images list to the user.

Advantageously, the assessment method of the present invention allows to automatically select or validate a collection of good quality images. For good quality herein is intended that the images comprise the necessary prerequisites (i.e. presence of fetal anatomical landmarks), according to medical guidelines.

According to one embodiment, the fetal organ is the fetal heart. In one alternative embodiment, the fetal organ is the fetus heart, brain, head (i.e. comprising the face) and or body.

According to one embodiment, the image analysis structure DL1 comprises a first stage employing a convolutional neural network. According to one embodiment, the first classifier of the image analysis structure DL1a comprises a second stage employing a fully connected neural network receiving as input at least a portion of the output of the first stage of the image analysis structure DL1.

According to one embodiment, when the valid images list comprises all the predefined required views of the fetal organ, the method is further configured to providing a message so as to inform the user that the valid images list comprises all the predefined required views of the fetal organ.

Advantageously, a doctor may use the valid images in the evaluation of fetuses examined as part of routine prenatal care [AIUM2003, ACR2003] in order to maximize the possibility of detecting a large spectrum of fetal abnormalities [Lee1998]. The valid ultrasound images allow as well to identify fetuses at risk for genetic syndromes and to provide useful information for patient counseling, obstetric management and multidisciplinary care.

According to one embodiment, whenever at least one image has been provided by the user manually, proving the image as input to the first classifier of the image analysis structure DL1a and the second classifier of the image analysis structure DL1b and whenever the first classifier DL1a identifies that the image corresponds to one view's category of the predefined list of view's categories and the second classifier DL1b identifies that a predefined number of fetal anatomical landmarks associated to the identified view's category are present in the image, adding said image to a valid images list.

According to one embodiment, whenever at least one image has been provided by the user manually, but not validated by the second classifier DL1b, proving the image as input to an object detector DL1c of the image analysis structure DL1 comprising a fourth stage configured to receive as input at least a portion of the output of the first stage of the image analysis structure DL1 and comprising region-based fully convolutional neural network architecture being configured to perform segmentation of the image, so as to classify and localize fetal anatomical landmarks in the image.

Fetal anatomical landmarks localization is computationally costly. In order to be realized in real time it would be needed to either use a high computational power or to simplify the deep learning algorithm and thus to accept to lose in accuracy of the numerical analysis. In order to overcome these two disadvantages, i.e. to be able to obtain high accuracy on a standard processor, the image analysis structure is divided into two mains architectures: (1) a first architecture comprising the first classifier DL1a and the second classifier DL1b configured to analyze the images of the fetal examination in real-time (neural networks treat each image that the user obtains with the ultrasound device) and (2) a second architecture comprising the object detector DL1c configured to analyze in non-real-time the images provided from the first architecture (DL1a and DL1b). This division strategy advantageously allows to obtain a list of valid images in real-time with an excellent precision and, if needed, segment them in non-real-time by keeping an excellent precision.

According to one embodiment, the predefined list of view's categories comprises ultrasound view categories from medical ultrasound guidelines [Carvalho2013, Lee2013, CNEOF2016].

According to one embodiment, the predefined fetal anatomical landmarks comprise physiological fetal landmarks from medical guidelines.

According to one embodiment, the user provides as input to the first classifier of the image analysis structure DL1a at least one image manually selected by the user from the ultrasound video sequence.

According to one embodiment, the method further comprises automatically generating and providing a report comprising an examination analysis based on the outputs of the image analysis structure DL1. Said report comprises information on:
- correspondence of the ultrasound images to the guideline views (i.e. output of the DL1a and DL1b classifiers), and if ultrasound images were provided manually, list and positions of the present anatomical landmarks as well as the list of the missing landmarks (i.e. output of the DL1c algorithm);
- information regarding whether all the predefined required views of the fetal organ were obtained or not.

According to one embodiment, when the valid images list comprises all the predefined required views of the fetal organ, the method of the present invention further comprises:
- providing a stack of at least one image of the valid images list as input to a diagnostic structure DL2, wherein the diagnostic structure DL2 comprises a first stage employing a convolutional neural network receiving as input the stack of images and providing an output and wherein said diagnostic structure DL2 comprises a first classifier DL2a employing, at a second stage, a fully connected neural network receiving as input at least a portion of the output of the first stage of the diagnostic structure DL2; the first classifier of the diagnostic structure DL2a being configured to discriminate between pathological development and physiological development of the fetal organ;
- whenever the output of the first classifier of the diagnostic structure DL2a categorizes the image as comprising a pathological development, proving the image as input to:
   o a second classifier DL2b of the diagnostic structure DL2 comprising a third stage employing a fully connected neural network receiving as input at least a portion of the output of the first stage of the diagnostic structure DL2 and being configured to classify the pathological development into at least one pathology category;
   o an object detector DL2c of the diagnostic structure DL2 comprising a fourth stage configured to receive as input at least a portion of the output of the first stage of the diagnostic structure DL2 and comprising a fully convolutional neural network being configured to perform segmentation of the image and localization of at least one pathological development region in the fetal organ;
   o providing as output to the user the at least one pathology category obtained from the second classifier DL2b and the result of the image segmentation of the image and localization of the pathological development region in the fetal organ obtained from the object detector DL2c of the diagnostic structure DL2;
- whenever the output of the first classifier of the diagnostic structure DL2a categorizes the images of the valid list of images as comprising a physiological development, proving to the user a message to interrupt examination.

This embodiment advantageously allows to evaluate the content of the ultrasound images in the valid image list so as to provide a diagnostic suggestion to the user.

According to one embodiment, the fully convolutional neural network of the fourth stage of the object detector DL2c is based on the region-based fully convolutional neural network architecture.

According to one embodiment, the method further comprises automatically generating and providing a report comprising an examination analysis based on the outputs of the image analysis structure DL1 and a diagnosis based on the outputs of the diagnostic structure DL2. Said report comprises information on:
- correspondence of the ultrasound images to the guideline views (i.e. output of the DL1a and DL1b classifiers), and if ultrasound images were provided manually, list and positions of the present anatomical landmarks as well as the list of the missing landmarks;
- information regarding whether all the predefined required views of the fetal organ were obtained or not;
- presence or absence of abnormal conditions in the fetus (i.e. output of the first classifier DL2a), and if fetal at least one abnormal condition is detected, a classification of this condition (i.e. output of the second classifier DL2b), as well as its localization on ultrasound images (i.e. output of the object detector DL2c).

According to one embodiment, the first and second classifier, and the object detector of the diagnostic structure DL2 are configured to receive as input a stack of images comprising at least one image.

According to one embodiment, the first stage convolutional neural networks of the image analysis structure and of the diagnostic structure have at least one common layer, defined during training.

According to one embodiment, the image analysis structure and of the diagnostic structure results from a simultaneous training, notably semi-supervised.

The present invention further relates to a computer program product for guiding a user in ultrasound assessment of a fetal organ, the computer program product comprising instructions which, when the program is executed by a computer, cause the computer to automatically carry out the steps of the method according to any one of the embodiments described above.

The present invention further relates to a computer readable storage medium for guiding a user in ultrasound assessment of a fetal organ comprising instructions which, when the program is executed by a computer, cause the computer to carry out the steps of the method according to any one of the embodiments described above.

The present invention also relates to a computer-implemented method for guiding a user in ultrasound assessment of a fetal organ so as to perform a diagnostic evaluation of the fetal organ development during a medical examination, said method comprising:
- receiving a valid images list wherein each image of said list has been obtained from an ultrasound images sequence comprising a predefined number of required views of the fetal organ;
- providing a stack of at least one image of the valid images list as input to a diagnostic structure DL2, wherein the diagnostic structure DL2 comprises a first stage employing a convolutional neural network receiving as input the stack of images and providing an output and wherein said diagnostic structure DL2 comprises a first classifier DL2a employing, at a second stage, a fully connected neural network receiving as input at least a portion of the output of the first stage of the diagnostic structure DL2; the first classifier of the diagnostic structure DL2a being configured to discriminate between pathological development and physiological development of the fetal organ;
- whenever the output of the first classifier of the diagnostic structure DL2a categorizes the image as comprising a pathological development, proving the image as input to:
   o a second classifier DL2b of the diagnostic structure DL2 comprising a third stage employing a fully connected neural network receiving as input at least a portion of the output of the first stage of the diagnostic structure DL2 and being configured to classify the pathological development into at least one pathology category;
   o an object detector DL2c of the diagnostic structure DL2 comprising a fourth stage configured to receive as input at least a portion of the output of the first stage of the diagnostic structure DL2 and comprising a fully convolutional neural network being configured to perform segmentation of the image and localization of at least one pathological development region in the fetal organ;
   o providing as output to the user the at least one pathology category obtained from the second classifier DL2b and the result of the image segmentation of the image and localization of the pathological development region in the fetal organ obtained from the object detector DL2c of the diagnostic structure DL2;
- whenever the output of the first classifier of the diagnostic structure DL2a categorizes the image as comprising a physiological development, proving to the user a message to end examination.

According to one embodiment, the fully convolutional neural network of the fourth stage of the object detector DL2c is based on the region-based fully convolutional neural network architecture.

According to one embodiment, the method further comprises automatically generating and providing a report comprising a diagnosis based on the outputs of the diagnostic structure DL2. Said report comprises information on presence or absence of abnormal conditions in the fetus (i.e. output of the first classifier DL2a), and if fetal at least one abnormal condition is detected, a classification of this condition (i.e. output of the second classifier DL2b), as well as its localization on ultrasound images (i.e. output of object detector DL2c).

According to one embodiment, the first and the second classifier and the object detector of a diagnostic structure DL2 are configured to receive as input a stack of images comprising at least one image and at the maximum limit the number of views required by the medical guidelines.

The present invention further relates to a computer program product for guiding a user in ultrasound assessment of a fetal organ so as to perform a diagnostic evaluation of the fetal organ development during a medical examination, the computer program product comprising instructions which, when the program is executed by a computer, cause the computer to automatically carry out the steps of the method according to any one of the embodiments described above.

The present invention further relates to a computer readable storage medium for guiding a user in ultrasound assessment of a fetal organ so as to perform a diagnostic evaluation of the fetal organ development during a medical examination, said computer readable storage medium comprising instructions which, when the program is executed by a computer, cause the computer to carry out the steps of the method according to any one of the embodiments described above.

The present invention also relates to a computer-implemented method for guiding a user in ultrasound assessment of a fetal organ so as to perform a diagnostic evaluation of the fetal organ development during a medical examination, said method comprising:
**In** what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. **In** addition, the modules are implemented in hardware, software, firmware, or any mixed form thereof as well.

The present invention also relates to a system for guiding a user in ultrasound assessment of a fetal organ so as to perform a diagnostic evaluation of the fetal organ development during a medical examination, said method comprising:
- input module configured to receive a valid images list, wherein each image of said list has been obtained from an ultrasound images sequence comprising a predefined number of required views of the fetal organ;
- a diagnostic module configured to:
   - provide a stack of one image of the valid images list as input to a diagnostic structure DL2, wherein the diagnostic structure DL2 comprises a first stage employing a convolutional neural network receiving as input the stack of images and providing an output and wherein said diagnostic structure DL2 comprises a first classifier DL2a employing, at a second stage, a fully connected neural network receiving as input at least a portion of the output of the first stage of the diagnostic structure DL2; the first classifier of the diagnostic structure DL2a being configured to discriminate between pathological development and physiological development of the fetal organ;
   - whenever the output of the first classifier of the diagnostic structure DL2a categorizes the image as comprising a pathological development, proving the image as input to:
      o a second classifier DL2b of the diagnostic structure DL2 comprising a third stage employing a fully connected neural network receiving as input at least a portion of the output of the first stage of the diagnostic structure DL2 and being configured to classify the pathological development into at least one pathology category;
      o an object detector DL2c of the diagnostic structure DL2 comprising a fourth stage configured to receive as input at least a portion of the output of the first stage of the diagnostic structure DL2 and comprising a fully convolutional neural network being configured to perform segmentation of the image and localization of at least one pathological development region in the fetal organ;
- output module configured to:
   - output to the user the at least one pathology category obtained from the second classifier DL2b and the result of the image segmentation of the image and localization of the pathological development region in the fetal organ obtained from the object detector DL2c of the diagnostic structure DL2;
   - output to the user a message to end the examination, whenever the output of the first classifier of the diagnostic structure DL2a categorizes the image as comprising a physiological development.

According to one embodiment, in the diagnostic module, the fully convolutional neural network of the fourth stage of the object detector DL2c is based on the region-based fully convolutional neural network architecture.

According to one embodiment, the output module is further configured to automatically generating a report comprising a diagnosis based on the outputs of the diagnostic structure DL2. Said report comprises information on presence or absence of abnormal conditions in the fetus (i.e. output of the first classifier DL2a), and if fetal at least one abnormal condition is detected, a classification of this condition (i.e. output of the second classifier DL2b), as well as its localization on ultrasound images (i.e. output of the object detector DL2c).

According to one embodiment, in the diagnostic module, the first and second classifier and the object detector of a diagnostic structure DL2 are configured to receive as input a stack of images comprising at least one image.

The present invention relates as well to a system for guiding a user in ultrasound assessment of a fetal organ so as to perform a diagnostic evaluation of the fetal organ development, based on an ultrasound images sequence comprising multiples predefined required views of the fetal organ, said system comprising:
- reception module configured to receive in real time a sequence of 2D ultrasound images acquired by the user, each image comprising at least a portion of the fetal organ;
- image analysis module configured to:
   o provide each image as input to an image analysis structure DL1 comprising at least one first classifier DL1a, said first classifier DL1a being configured to identify if the image belongs to any view's category comprised in a predefined list of view's categories and, if so, to identify the view's category to which belongs the image among said predefined list of view's categories; wherein each view's category is associated to at least one predefined fetal anatomical landmark;
   o provide each image as input to a second classifier DL1b of the image analysis structure DL1, said second classifier DL1b being configured to detect the presence in the image of predefined fetal anatomical landmarks;
   o whenever the first classifier DL1a identifies that the image corresponds to one view's category of the predefined list of view's categories and the second classifier DL1b identifies that a predefined number of fetal anatomical landmarks associated to the identified view's category are present in the image, adding said image to a valid images list;
   o providing the valid images list.

According to one embodiment, in the image analysis module, the image analysis structure DL1 comprises a first stage employing a convolutional neural network and wherein the first classifier of the image analysis structure DL1a comprises a second stage employing a fully connected neural network receiving as input at least a portion of the output of the first stage of the image analysis structure DL1.

According to one embodiment, the image analysis module is further configured to provide a message to inform the user that the valid images list comprises all the predefined required views of the fetal organ, when the valid images list comprises all the predefined required views of the fetal organ.

According to one embodiment, the system further comprises a manual input module configured to receive images provided manually as input by the user which were selected from the ultrasound video sequence.

According to one embodiment, whenever at least one image has been provided by the user manually, the image analysis module is further configured to provide the image as input to the first classifier of the image analysis structure DL1a and the second classifier of the image analysis structure DL1b and whenever the first classifier DL1a identifies that the image corresponds to one view's category of the predefined list of view's categories and the second classifier DL1b identifies that a predefined number of fetal anatomical landmarks associated to the identified view's category are present in the image, adding said image to a valid images list.

According to one embodiment, whenever at least one image has been provided by the user manually and but not validated by the second classifier DL1b, the image analysis module is further configured to provide the image as input to an object detector DL1c of the image analysis structure DL1 comprising a fourth stage configured to receive as input at least a portion of the output of the first stage of the image analysis structure DL1 and comprising region-based fully convolutional neural network architecture being configured to perform segmentation of the image, so as to classify and localize fetal anatomical landmarks in the image.

According to one embodiment, the system of the present invention further comprises:
- a diagnostic module configured to, when the valid images list comprises all the predefined required views of the fetal organ:
   - provide a stack of one image of the valid images list as input to a diagnostic structure DL2, wherein the diagnostic structure DL2 comprises a first stage employing a convolutional neural network receiving as input the stack of images and providing an output and wherein said diagnostic structure DL2 comprises a first classifier DL2a employing, at a second stage, a fully connected neural network receiving as input at least a portion of the output of the first stage of the diagnostic structure DL2; the first classifier of the diagnostic structure DL2a being configured to discriminate between pathological development and physiological development of the fetal organ;
   - whenever the output of the first classifier of the diagnostic structure DL2a categorizes the image as comprising a pathological development, proving the image as input to:
      o a second classifier DL2b of the diagnostic structure DL2 comprising a third stage employing a fully connected neural network receiving as input at least a portion of the output of the first stage of the diagnostic structure DL2 and being configured to classify the pathological development into at least one pathology category;
      o an object detector DL2c of the diagnostic structure DL2 comprising a fourth stage configured to receive as input at least a portion of the output of the first stage of the diagnostic structure DL2 and comprising a fully convolutional neural network being configured to perform segmentation of the image and localization of at least one pathological development region in the fetal organ;
- output module configured to:
   - output to the user at least one pathology category obtained from the second classifier DL2b and the result of the image segmentation of the image and localization of the pathological development region in the fetal organ obtained from the object detector DL2c of the diagnostic structure DL2;
   - output to the user a message to end the examination, whenever the output of the first classifier of the diagnostic structure DL2a categorizes the image as comprising a physiological development.

According to one embodiment, in the diagnostic module, the fully convolutional neural network of the fourth stage of the object detector DL2c is based on the region-based fully convolutional neural network architecture.

According to one embodiment, the output module is further configured to automatically generating a report comprising an examination analysis based on the outputs of the image analysis structure DL1 and a diagnosis based on the outputs of the diagnostic structure DL2. Said report comprises information on:
- correspondence of the ultrasound images to the guideline views (i.e. output of the DL1a and DL1b classifiers), and if ultrasound images were provided manually, list and positions of the present anatomical landmarks as well as the list of the missing landmarks (i.e. output of the DL1c algorithm);
- information regarding whether all the predefined required views of the fetal organ were obtained or not;
- presence or absence of abnormal conditions in the fetus (i.e. output of the first classifier DL2a), and if fetal at least one abnormal condition is detected, a classification of this condition (i.e. output of the second classifier DL2b), as well as its localization on ultrasound images (i.e. output of the object detector DL2c).

According to one embodiment, in the diagnostic module, the first and second classifier and the object detector of the diagnostic structure DL2 are configured to receive as input a stack of images comprising at least one image.

According to one embodiment, the first stage convolutional neural networks of the image analysis structure and of the diagnostic structure have at least one common layer, defined during training.

According to one embodiment, the image analysis structure and of the diagnostic structure results from a simultaneous training, notably semi-supervised.

### DEFINITIONS

In the present invention, the following terms have the following meanings:
- **"Database"** refers to collections of data used to build a machine learning (ML) mathematical model, so as to make data-driven predictions or decisions. In supervised learning (i.e. inferring functions from known input-output examples in the form of labelled training data), three types of ML datasets (also designated as ML sets) are typically dedicated to three respective kinds of tasks: training, i.e. fitting the parameters, validation, i.e. tuning ML hyperparameters (which are parameters used to control the learning process), and testing, i.e. checking independently of a training dataset exploited for building a mathematical model that the latter model provides satisfying results.
- **"Fetus"** and **"fetal"** refers to is the unborn offspring of a mammal, preferably a human that develops from an embryo.
- **"Convolutional neural network"** refers to a neural network which is partly composed of convolutional layers, i.e. layers which apply a convolution on their input. Said series of convolutional layers comprise filters (Kernal) applied to the input data, such as images, in order to automatically extract from them convoluted feature maps. Convolutional neural network may comprise as well different pooling layers, linear and non-linear activation functions and numerical technics (i.e. batch normalization, dropout, etc) for learning acceleration and stabilization. Very often, convolutional neural networks are connected with fully connected "dense" layers.

- **"Fully convolutional neural network"** refers to a convolutional neural network without any fully connected "dense" layers used in the architecture.
- **"Neural network"** refers to a mathematical structure taking an object as input and producing another object as output though a set of linear and non-linear operations called layers. Such structures have parameters which can be tuned through a learning phase so as to produce a particular output, and are for instance used for classification purposes. The input is then the object to categorize, and the output is an estimation of at least a part of the characteristics of the input object.
- **"Processor",** this term is herein not restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.
- **"Real time":** refers to the ability of a system of controlling an environment by receiving data, processing them, and returning the results sufficiently quickly to affect the environment at that time. Real-time responses (i.e. output) are often understood to be in the order of milliseconds, and sometimes microseconds.

### BRIEF DESCRIPTION OF THE DRAWINGS

Features and advantages of the invention will become apparent from the following description of embodiments of a system, this description being given merely by way of example and with reference to the appended drawings in which:
**Figure 1** schematic representation of a list of some of the ultrasound view categories required by the ISUOG OB/GYN guidelines for the fetal heart examination of the 2^{nd} and 3^{rd} trimesters of pregnancy.
**Figure 2** is an example of a valid four-chamber view of a fetal heart representing all the fetal anatomical landmarks associated with this view and required by the OB/GYN guidelines.
**Figure 3** is an example of a non-valid four-chamber view missing some of the important fetal anatomical landmarks required for a correct diagnosis and thus not corresponding to the OB/GYN medical guidelines.
**Figure 4** is a flow chart showing the steps of the method for the analysis of the ultrasound images sequence and generation of a valid images list.
**Figure 5** is schematic representation of the image analysis structure DL1(a-c) configured to validate the correspondence of the fetal ultrasound images to the guideline views.
**Figure 6** is a flow chart showing the main steps of the method for guiding a user in ultrasound assessment of a fetal organ so as to perform a diagnostic evaluation of the fetal organ development during a medical examination according to one embodiment of the present invention.
**Figure 7** is a flow chart showing the steps of the method concerning analysis of invalid images to obtain information on the present and absent fetal anatomical landmarks.
**Figure 8** is a schematic presentation of the deep learning architecture DL1c that aims to determine and localize the fetal anatomical landmark on the fetal ultrasound images.
**Figure 9** is a schematic presentation of the deep learning architecture DL2.
**Figure 10** is an example of output provided to the user when some view's categories have still not been acquired.
**Figure 11** is an example of output provided to the user to inform that all predefined view's categories have been acquired, all of them satisfying the guideline views and no pathological development was detected.
**Figure 12** is an example of output provided to the user to inform that all predefined view's categories have been acquired, all of them satisfy the guideline views, but a pathological development was detected in some of the ultrasound images.
**Figure 13** is an example of output concerning ultrasound images provided manually by user, in a case wherein one image does not comprise the required fetal anatomical landmarks corresponding to the view's category to which it belongs and thus does not satisfy to the guidelines.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

### DETAILED DESCRIPTION

The following detailed description will be better understood when read in conjunction with the drawings. For the purpose of illustrating, the method's steps are shown in the preferred embodiments. It should be understood, however that the application is not limited to the precise arrangements, structures, features, embodiments, and aspect shown. The drawings are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

The present invention relates to a computer-implemented method for guiding a user in ultrasound assessment of a fetal organ so as to assess the quality of a medical examination. The present computer-implemented method receives as input and perform calculation on an ultrasound images sequence comprising multiples predefined required views of the fetal organ.

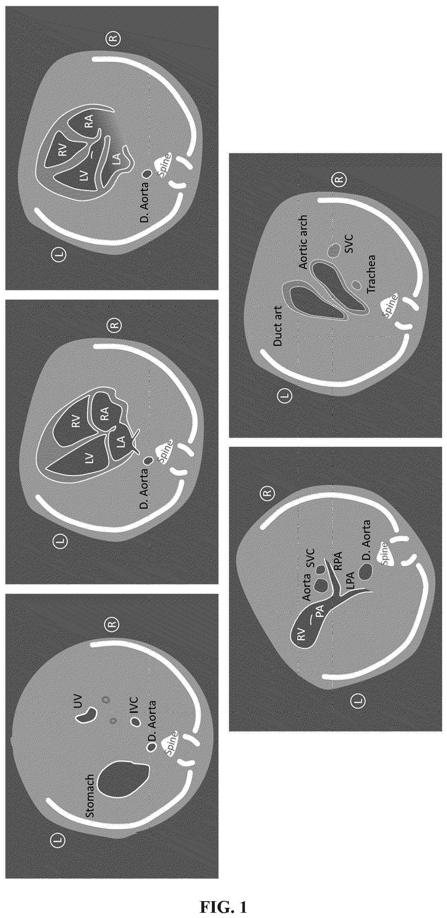

**In** one embodiment, the system is configured to provide to the user a list of some of the ultrasound views categories required, for example by the ISUOG OB/GYN guidelines, for the fetal heart examination of the 2^{nd} and 3^{rd} trimesters of pregnancy. Some of these ultrasound views categories required are represent in Figure 1.

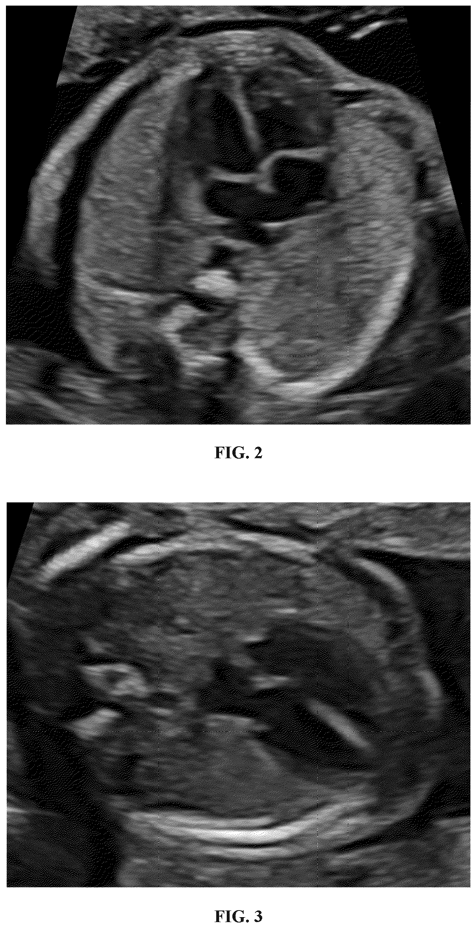

An ultrasound view of a specific fetal organ is an ultrasound image comprising a standardized list of visible fetal anatomical landmarks of such fetal organ. For example, in a valid four-chamber view it should be visible the whole heart inside the chest, the size of the picture should take at least half of the screen, and the proportion of the heart, in normal condition, compared to the chest is usually 1/3. Furthermore, in a four-chamber view it should be visible the following fetal anatomical landmarks: the apex of the heart, both ventricles if present, the atrio-ventricular valves and their offset, both atria, at least one pulmonary vein, the foramen ovale, and the vieussens valve. An example of a such valid four-chamber view, corresponding to OB/GYN guidelines, is shown in Figure 2.

Alternatively, a non-valid view of fetal examination is a view that misses at least one of the important fetal anatomical landmarks required for a correct diagnosis. As example, the four-chamber view demonstrated in Figure 3 doesn't allow to assess thoroughly the atrio-ventricular valves and their offset. This failure exposes the operator to misrecognition of inlet abnormalities such as atrio-ventricular septal defect (AVSD) or tricuspid valve abnormalities such as Epstein disease and tricuspid dysplasia.

Another example may concern the fetal profile. In a valid view corresponding to OB/GYN guidelines, it is mandatory to be able to display the forehead, the nose, the upper and the lower lips, and the chin. If at least one of these landmarks is missing, it will not be possible to properly assess the fetal profile.

A real challenge for AI is, at first, to be able to distinguish high-quality from low-quality views, at second, to differentiate abnormal from normal low-quality views, and finally, to identify abnormal from normal views. Indeed, in all of these cases some of these landmarks can be missing. When an ultrasound image comprises all the fetal anatomical landmarks of the standardized list associated to a specific view then the image belongs to a category of images representing said specific view of the fetus. An image belonging to a view category of a fetal organ comprises at least a part of information necessary for the correct evaluation of the fetal organ, and this whatever the normal or abnormal condition of the organ. Usually, multiple images belonging to different views' categories of a fetal organ are necessary to evaluate the fetal organ and provide a correct diagnosis. Said views' categories are defined in medical ultrasound guidelines.

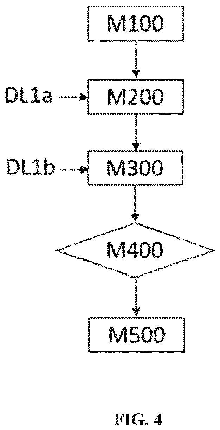

Figure 4 shows the main steps of this computer-implement method for guiding a user in ultrasound assessment of a fetal organ so as to assess the quality of an examination. No diagnosis is determined in the routine represented by the steps of Figure 4. According to one embodiment, the method comprises a step M100 of receiving a sequence of 2D ultrasound images comprising multiples predefined required views of the fetal organ, each image comprising at least a portion of the fetal organ. These images may have been previously acquired by the user. Alternatively, the user performs a fetal ultrasound examination in real time resulting in a sequence of 2D ultrasound images which are therefore received in real time.

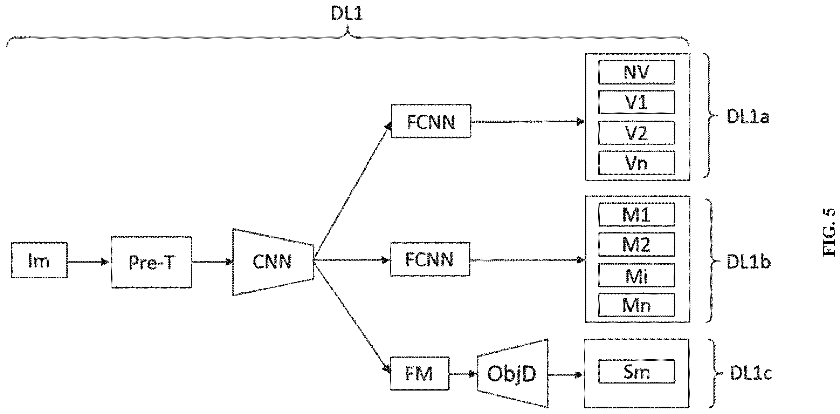

According to one embodiment, the method comprises a step M200 providing each image as input to an image analysis structure DL1. DL1 is a deep learning structure configured to validate the correspondence of the fetal ultrasound images to medical guidelines. A schematic representation of the image analysis structure DL1 according to one embodiment of the invention is shown in Figure 5. In this Figure the box "Im" represents the image inputted in the DL1 structure.

According to one embodiment, the image analysis structure DL1 comprising at least one first classifier DL1a which is configured to identify if the image belongs to any view's category comprised in a predefined list of view's categories and, if so, to identify the view's category to which belongs the image among said predefined list of view's categories. As explained above, each view's category is associated to at least one predefined fetal anatomical landmark. Advantageously, the quality of each image is evaluated by the presence and/or the absence of specific predefined fetal anatomical landmarks and display criteria. In this case, even with poor signal-to-noise ratio (i.e. a lot of noise for example), but all necessary fetal anatomical landmarks present, the image analysis and further diagnosis will be possible.

According to one embodiment, the predefined list of view's categories comprises ultrasound view categories from medical ultrasound guidelines.

According to one embodiment, the predefined fetal anatomical landmarks comprise physiological fetal landmarks from medical guidelines.

According to one embodiment, the image analysis structure DL1 comprises a first stage employing a convolutional neural network (CNN). Advantageously, CNN architecture shown its efficiency for the tasks related to computer vision and image treatment as a very powerful and efficient model performing automatic feature extraction to achieve superhuman accuracy. More in details, CNN architecture presents many advantages compared with its predecessors. The filters of CNNs are designed to automatically extract and learn important features from the images. CNN architectures treat directly 2D and 3D data which could be gray-scale and color images and are much more computationally efficient (in terms of number of trainable parameters, weights, memory etc.). As a result, the CNN architectures provide higher prediction accuracy on tasks related to computer vision, image/videos treatments, etc.

The image analysis structure DL1 may further comprise at said first stage bias layer, max pooling layers, batch normalization and/or activation layers.

The first stage of DL1 may comprise, before the convolutional neural network, a pre-processing the image, for example for denoising the image.

According to one embodiment, the first classifier of the image analysis structure DL1a comprises a second stage employing a fully connected neural network (FCNN) receiving as input at least a portion of the output of the first stage of the image analysis structure DL1.

In one example represented in Fig. 5, the first classifier of the image analysis structure DL1a is configured to provide as output, for each provided image, a softmax vector of size N+1 where N corresponds to the number of view categories (V1, V2, ..., Vn) and the additional one corresponds to the case when the category cannot be defined (NV). The category of each image is then determined using the argmax mathematical operation.

According to one embodiment, the first classifier of the image analysis structure DL1b comprises a second stage employing a fully connected neural network (FCNN) receiving as input at least a portion of the output of the first stage of the image analysis structure DL1.

According to one embodiment, the method further comprises a step M300 providing each image as input to a second classifier DL1b comprised in the image analysis structure DL1. Said second classifier DL1b is configured to detect the presence in the image of predefined fetal anatomical landmarks. Preferably, the second classifier DL1b may be configured to identify but not to localize the fetal anatomical landmarks present in the image.

In one example, for each provided image the second classifier DL1b provides as output a vector of size M, where M corresponds to the total number of fetal anatomical landmarks associated to the fetal organ. The fetal anatomical landmarks present on the provided image are defined using the following mathematical rule: *where(vector >* 0).

According to one embodiment, the method comprises a question step M400 consisting in evaluating whether or not the first classifier DL1a had identified that the image corresponds to one view's category of the predefined list of view's categories and, at the same time, the second classifier DL1b had identified that a predefined number of fetal anatomical landmarks associated to the identified view's category are present in the image.

According to one embodiment, the method comprises an updating step M500 consisting in adding the image to a valid images list if the first classifier DL1a had identified that the image corresponds to one view's category of the predefined list of view's categories and the second classifier DL1b had identified that a predefined number of fetal anatomical landmarks associated to the identified view's category are present in the image. All other images are discarded as non-valid. In other words, the steps M400 and M500, are configured to verify if a view of the predefined list of view's categories was acquired and if all the required fetal anatomical landmarks corresponding to this view were identified, if so the image is considered as valid to be used in future for the evaluation of a diagnosis.

According to one embodiment, the method comprises as well as step of providing the valid images list to the user.

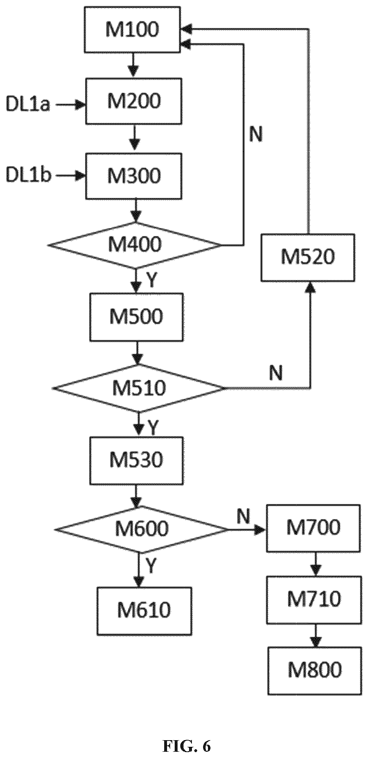

According to one embodiment, each time the valid images list is updated with a new valid image the method is configured to verify whether the valid images list comprises all the predefined required views of the fetal organ. This step is represented as M510 in Figure 6.

According to one embodiment, the method further comprises a step of providing a message to inform the user that the valid images list comprises all the predefined required views of the fetal organ, when the valid images list comprises all the predefined required views of the fetal organ. Furthermore, the method may as well comprise a step M520 of providing a message to inform the user that the valid images list still does not comprise all the predefined required views of the fetal organ and suggest to continue the examination by acquiring new image sequences. In this case the use may continue the examination and therefore acquire more sequences of 2D ultrasound images. These new acquired sequences of 2D ultrasound images are inputted in a processor and analyzed according to step of the method of the present invention as presented here above.

The user may have the option to provide the required images manually. In this case the use may select manually an image from the sequence of 2D ultrasound images which is believed to be conform to the guidelines (i.e. classic way of fetal examination today).

It may not be mandatory to specify which of the view categories this image belongs to, it will be done automatically by the DL1 algorithm, as described below.

According to one embodiment, whenever at least one image has been provided by the user manually, the method comprises proving the image as input to the first classifier of the image analysis structure DL1a and the second classifier of the image analysis structure DL1b. In this embodiment, whenever the first classifier DL1a identifies that this manually provided image corresponds to one view's category of the predefined list of view's categories and the second classifier DL1b identifies that a predefined number of fetal anatomical landmarks associated to the identified view's category are present in the image, adding said image to a valid images list.

According to one embodiment, the method further comprises, whenever at least one image has been provided by the user manually but not validated by the second classifier DL1b, the step M310 of providing the image as input to an object detector DL1c of the image analysis structure DL1 to classify and localize fetal anatomical landmarks that are present in the image. The object detector DL1c is configured to perform a fast segmentation of the image, classify and localize fetal anatomical landmarks that are present in the image.

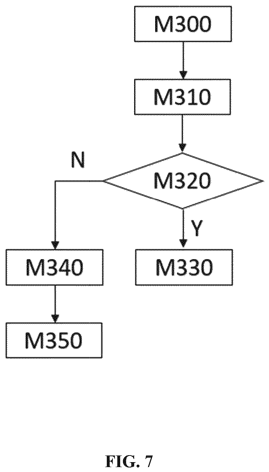

In one embodiment shown in Figure 7, the fetal anatomical landmarks detected by the second classifier DL1b at step M300 are compared to the ones identified by the object detector DL1c at step M310. If both structures have identified the same fetal anatomical landmarks (step M320), then these fetal anatomical landmarks are stored as a final set of the image landmarks (step M330). Inversely, if the fetal anatomical landmarks detected by the second classifier DL1b and the object detector DL1c are not de same, the method comprises using a different deep learning algorithm for segmentation which is applied as a judge-method (step M340) and the output provides the final set of the image landmarks (step M350).

In one embodiment, the final set of the image landmarks is provided to the use comprising information on the present and absent fetal anatomical landmarks. Advantageously, the method provides user-oriented information allowing to improve the examination quality.

If the images provided by the user manually are validate by the image analysis structure DL1, these images are adding said image to a valid images list.

According to one embodiment, the object detector DL1c may comprise a fourth stage configured to receive as input at least a portion of the output of the first stage of the image analysis structure DL1 and comprising region-based fully convolutional neural network architecture being configured to perform segmentation of the image.

In one example, for each provided image the object detector DL1c yields two different answers. For each region of interest found on the image, DL1c returns (M+1)-dimensional softmax responses across M fetal anatomical landmark (+1 for the background). The fetal anatomical landmark is then defined for each region of interest using the argmax mathematical operation. Similarly, for each region of interest found on the image, DL1c returns 4-dimentional average voting responses related to the bounding box and corresponding to the center position of the box along the x axis, center position of the box along the y axis, box width and box height, a 5th position corresponding to the box rotation angle may be added as well.

Advantageously the structure of the three neural networks (DL1a, DL1b, DL1c) are fast enough to be applied in real time image processing. However, only DL1a and DL1b classifier need to be applied in real time, whereas the object detector DL1c is not required for real-time analysis.

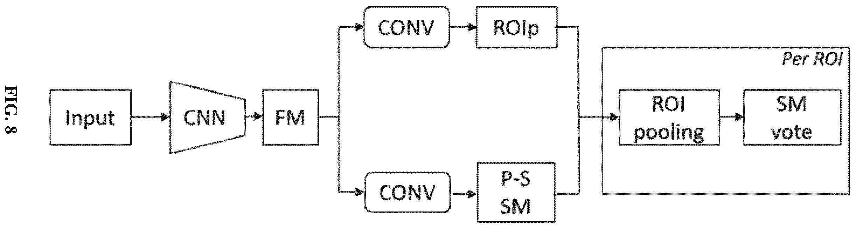

The architecture of object detector DL1c is presented in Fig. 8. According to the example of Figure 8, the realization of network DL1c is based on region-based fully convolutional neural network (R-FCN) architecture implemented and adapted to the ultrasound fetal examination. Alternatively, others deep learning architectures may be applicable such as the R-CNN, Fast R-CNN, Faster R-CNN, YOLO or SSD (Single Shot Detection).

The backbone of object detector DL1c architecture is unique to this invention and pre-trained on the problems of the classification of the view categories and fetal anatomical landmarks (DL1a and DL1b architectures respectively). Feature maps (referred to as FM in Fig. 8) are generated by some internal convolutional layers of the R-FCN, whereas the last convolutional layers generate the feature maps for DL1a, DL1b, DL2a and DL2b structures which afterword go to the FCNN architecture. Regions of Interest (RoI) are independent from the region-based feature maps. Construction of ROIs propositions is done using Region Proposal Network algorithm (referred to as ROIp in Fig. 8). Position-sensitive score maps (referred to as P-S SM in Fig. 8) represent a special type of the feature maps each detecting the corresponding sub-region of the anatomical landmark. Position-sensitive score maps are obtained through convolution operation of dimension *k*² (*C* + 1), where *k*² corresponds to the total size of the feature score maps, *C* + 1 is the number of possible fetal anatomical landmarks plus one class of background (non-landmark). ROI pooling is produced in a loop for each of the ROI found. Each layer is of size *k*². Finally, a softmax activation function (referred to as "SM vote" in Fig. 8) is applied in order to distinguish fetal anatomical landmarks from background and classify the fetal anatomical landmarks. The bounding box regression is shared among the classes and defines for each of the ROI four values: center position along the x axis, center position along the y axis, width and height. As it was previously mentioned, the 5^{th} value may be added in order to take into account the rotation angle of the box. The final number of fetal anatomical landmarks, that is being searched for the given view category, is known in advance. These fetal anatomical landmarks are unique and therefore provide an *a-priori* information about their location, size and orientation that are implemented into the Region Proposal Network algorithm.

According to one embodiment represent in Figure 6, when the valid images list comprises all the predefined required views of the fetal organ (step M530) the examination is validated and the method of the present invention further comprises a step M600 of evaluating whether the fetal development is physiological or not.

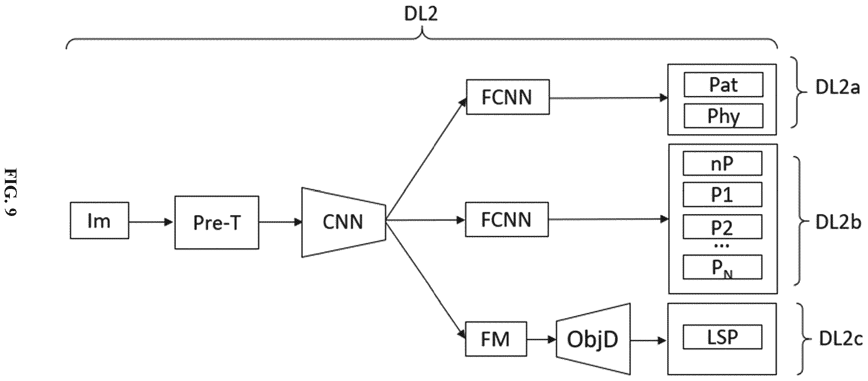

In one embodiment, this step M600 comprises providing a stack of at least one image of the valid images list as input to a diagnostic structure DL2. The deep learning structure DL2 is schematically represented in Figure 9. The maximum number of images for one input of the DL2 structure is equal to the number of predefined views required, for example, according to the guidelines in France. In one embodiment, the diagnostic structure DL2 comprises a first classifier DL2a, a second classifier DL2b and an object detector DL2c.

In one embodiment, the stack of images comprises one, two, three, four, five or more fetal ultrasound images simultaneously, wherein the number of images in the stack corresponds to Ni, the predefined number of required views of the fetal organ. This input is common for DL2a, DL2b and DL2c deep learning algorithms. This architecture accepts as well at least one ultrasound image, in order to analyze this given image for the presence of fetal abnormalities. In this case, N images (1<= N <= Ni) are placed in the stack with zeros layers for the missing images.

For example, a stack comprising one image may be used when the user wants to analyze only one image manually provided to check whether a given image is pathological or physiological.

In one preferred embodiment, the stack of images comprises at least two images. Advantageously taking into consideration multiple images (i.e. multiple views) at the same time provides much more useful information to the Neural Network structures and increases the detection rate. Indeed, for the detection of some heart (or brain for example) abnormalities, the user has to consider multiple organ views at the same time (example: physicians usually look at the abdominal situs view and 4 chambers view at the same time).

In one embodiment, the diagnostic structure DL2 comprises a first stage employing a convolutional neural network (CNN) receiving as input the stack of images and providing an output. Advantageously, the CNN structure guarantees a high precision. For the realization of this embodiment the object-oriented architectures are not well adapted (i.e. YOLO, SSD etc.). As shown in Figure 9, the stack of images ("Im") may be pre-treated before being provided as input to the convolutional neural network.

In one embodiment, the first classifier DL2a employs, at a second stage, a fully connected neural network receiving as input at least a portion of the output of the first stage of the diagnostic structure DL2. Said first classifier of the diagnostic structure DL2a is configured to discriminate between pathological development ("Pat") and physiological development ("Phy") of the fetal organ.

The method may comprise a step M610 of outputting to the user a message to end examination whenever the output of the first classifier of the diagnostic structure DL2a categorizes the image as comprising a physiological development.

In one example, independently on the number of the provided ultrasound images in the stack, the DL2a structure yields a vector of size two (i.e. two neurons). The activation of each of these neurons corresponds respectively to the physiological and pathological development. Output values of these neurons are normalized using the softmax activation function. Finally, an argmax mathematical operation is applied to the normalized vector in order to obtain the neuron containing the maximal value. The selected neuron will correspond to the physiological or pathological development.

In one embodiment, whenever the output of the first classifier of the diagnostic structure DL2a categorizes the image as comprising a pathological development, the method comprises the step M700 of proving the image as input to the second classifier DL2b of the diagnostic structure DL2.

In one embodiment, the second classifier DL2b comprises a third stage employing a fully connected neural network receiving as input at least a portion of the output of the first stage of the diagnostic structure DL2. The second classifier DL2b is configured to classify the pathological development into at least one pathology category Pi or an unknown pathology nP.

In one example, for each stack of images, the DL2b algorithm yields a vector of size (N+1) where N corresponds to the total number of the known pathologies (P1, P2, ..., PN) and the plus 1 is added to take into account the case of unknown pathology (nP). The pathologies represented on the provided stack of images are defined using the following mathematical rule: *where(vector >* 0).

In one embodiment, whenever the output of the first classifier of the diagnostic structure DL2a categorizes the image as comprising a pathological development, the method comprises as well proving the image as input to the object detector DL2c of the diagnostic structure DL2 (step M710). Said object detector DL2c comprises a fourth stage configured to perform segmentation of the image and localization of at least one pathological development region in the fetal organ.

In one embodiment, the object detector DL2c is configured to receive as input at least a portion of the output of the first stage of the diagnostic structure DL2. The fourth stage of the object detector DL2c may comprise a fully convolutional neural network configured to perform segmentation of the image and localization of at least one pathological development region in the fetal organ. The fully convolutional neural network of the fourth stage of the object detector DL2c may notably be a region-based fully convolutional neural network architecture.

In one embodiment, for each of the images in the stack, the object detector DL2c yields two different information.

The first information concerns the pathology category. More precisely the detector DL2c, for each region of interest found on each image, returns a (M+3)-dimensional softmax, wherein M corresponds to the number of known pathologies, one additional dimension (i.e. +1) is used to take into account an unknown pathology recognized by the object detector DL2c, one other dimension is used to account for physiological anatomy region of interests, and one dimension takes into account the background of the image (i.e. uterus). The classification is then defined for each region of interest of each of the images of the stack using the argmax mathematical operation.

Another embodiment of the DL2c architecture, not represented, may also be implemented. In this embodiment, for the first information, the object detector DL2c returns only a 3-dimensional softmax response across: one physiological case, one pathological case and a background.

Advantageously, this embodiment allows a simpler implementation and the obtention of more precise results. However, it does not allow to classify abnormalities present in the region of interests. In other words, the classifier is able to identify whether the anatomical parts of the fetus in the ROI are physiological, pathological or belongs to a background (i.e. not physiological or pathological). But in the case if a pathology found, the classifier is not able to classify the anatomical parts of the fetus and precisely determine which pathology is present.

The second information concerns the result of the image segmentation of the images of the stack and localization of the pathological development region in the fetal organ. For each region of interest found on each image in the provided stack, the object detector DL2c returns 4-dimentional average voting responses related to the bounding box and corresponding to the center position of the box along the x axis, center position of the box along the y axis, box width and box height (output referred to as "LSP" in Fig. 9). A fifth position corresponding to the box rotation angle may be added as well.

In one embodiment, the method also comprises providing an output to the user (step M800). This output may be the at least one pathology category obtained from the second classifier DL2b and the result of the image segmentation of the image and localization of the pathological development region in the fetal organ obtained from the object detector DL2c of the diagnostic structure DL2.

According to one embodiment, whenever the output of the first classifier of the diagnostic structure DL2a categorizes all the images comprised in the valid list of images as comprising a physiological development, the method is configured to prove to the user a message to interrupt the examination. In this embodiment, we refer to the valid images list comprising all the predefined required views of the fetal organ.

In one embodiment, the image analysis structure and the diagnostic structure have some common convolutional layer in the Encoder (i.e. the first stage convolutional neural networks of the image analysis structure and of the diagnostic structure). The common layers may be defined during the training part through an optimization technique. An example of such application is a particle swarm optimization method applied to a level set approach. This approach results in a simultaneous training of architectures DL1 and DL2 through the transfer learning technique. Advantageously, training the two structures at the same time allows to yield better results that two separate structures trained independently.

In one embodiment, the image analysis structure and the diagnostic structure results are obtained by a simultaneous training, notably semi-supervised.

In one embodiment, the fully connected neural networks of DL1a and DL2a comprise at least one hidden layer.

In one embodiment, the fully connected neural networks of DL1b and DL2b comprise at least two hidden layers.

In one embodiment, DL1c and DL2c object detector are fully convolutional and do not comprise any fully connected layers.

For example, in case of classification task, a binary cross entropy loss function is applied to a softmax activation layer for the both DL1 and DL2 neural architectures. In case of object detection task, an adapted loss function *L*(*s, t_{x,y,w,h,θ}*) = *L_{cls}*(*s*) *+* [≠ *b*] *L_{reg}*(*t, t**) is implemented, where *L_{cls}*(*s*) is a cross entropy loss function for the classification task, *L_{reg}*(*t, t**) is a regression loss function for the bounding box search task, and [≠ *b*] determines the case when the ground-truth label of the Region of Interest does not correspond to the background. A parameter *θ* responsible for the box rotation angle is added to t in order to improve the precision of fetal landmarks search. Finally, a binary cross entropy loss function is used for the implementation of a Generative Adversarial Network (GAN) as a semi-supervised approach. This loss function takes the output of the supervised model prior to the softmax activation function and applies a special activation function calculating a normalized sum of the exponential outputs. A stochastic gradient descent algorithm Adam is used for training of this hybrid architecture with momentum values of 0.5-0.9 and learning rate value of 2 * 10⁻⁴. Another approach may be implemented which is based on the idea of creating a neuron independent from the supervised model for the GAN architecture. The designing an optimal neural architecture can be very challenging in terms of computational requirements. A numerical optimization algorithm is thus implemented in order to choose neural network parameters leading to an optimal architecture. This algorithm is based on a level-set approach for optimizing discrete parameters and the metamodeling technique allowing to build surrogate models to expensive black-box functions and to reduce the computational burden for optimization purpose.

In one embodiment, the image analysis structure DL1 (i.e DL1a, DL1b and DL1c) is trained using a database containing both physiological and pathological images. It is done with a purpose to learn the image analysis structure DL1 to distinguish fetal abnormalities from the missing fetal landmarks on the image.

In one embodiment, the method is a software on cloud solution to which a user may access through an internet connection.

The present invention also relates to the system comprising a computer storage medium and a processor configured to carry out the method of the present invention.

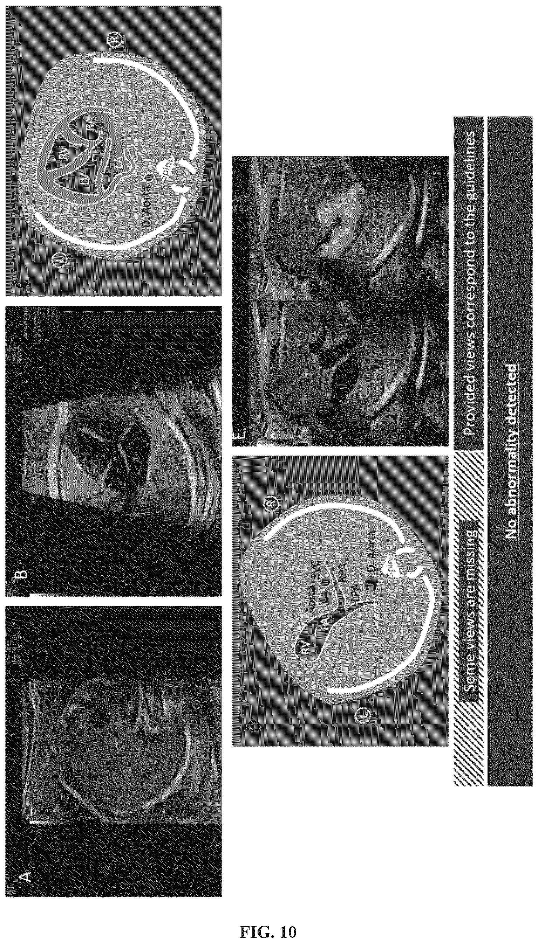

To provide an illustrative example of the invention in the case of a fetal heart, some of the provided outputs are presented in Figures 10 to 13.

First of all, as disclosed above, the user has access to a list of some of the ultrasound view categories required, for example by the ISUOG OB/GYN, guidelines for the fetal heart examination of the 2^{nd} and 3^{rd} trimesters of pregnancy (Fig. 1).

The user starts the examination with the ultrasound device knowing that a sequence of ultrasound images should be obtained wherein at least a part of the images is comprised in this list of ultrasound view categories.

During fetal examination (i.e. acquisition of the ultrasound images sequence), the first classifier DL1a and the second classifier DL1b of the image analysis structure run in order to select the good images of the fetal organ (i.e. images corresponding to one view's category of the predefined list of view's categories and comprising a predefined number of fetal anatomical landmarks associated to the identified view's category) and add them into the list of valid images. The user may be informed that some views are still missing from a graphic representation like in Figure 10. Indeed, in Figure 10 the left ventricular outflow tract (LVOT) view (drawing in Fig. 10C) and right ventricular outflow tract (RVOT) view (drawing in Fig. 10D) have not been acquired yet, while images of the abdominal situs ascertained in a transverse view of the fetal abdomen (ultrasound image in Fig. 10A), four-chamber view (ultrasound image in Fig. 10B) and three vessels and trachea (3VT) view have been successfully acquired. At this point of the examination no abnormality is detected.

Once the list of valid images selected by the first classifier DL1a and the second classifier DL1b comprises at least one image for each of the predefined list of view's categories, the invention provides an output to the user to inform him/her that the examination is "valid" (i.e. the valid images list comprises all the predefined required views of the fetal heart and these images therefore correspond to the guidelines).

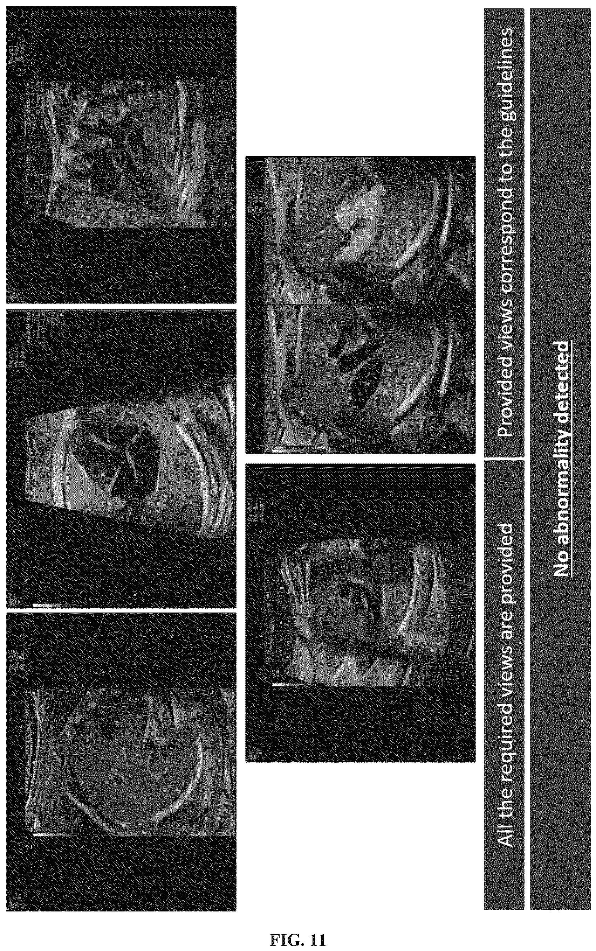

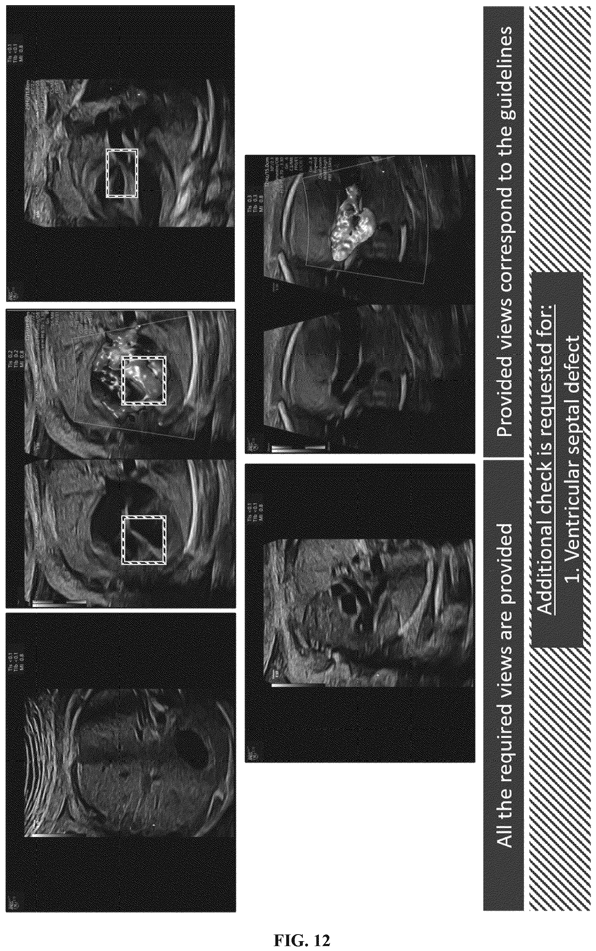

The images of the valid images list are treated with the first classifier of the diagnostic structure DL2a in order to distinguish physiological development from the pathological one. In case of physiological development of the fetus heart, the system/method of the invention provides an output to the user such as the one illustrated in Figure 11. In this case a written message informs the user that no abnormality is detected. However, if a pathology is detected by first classifier DL2a, then classified into at least one pathology category by DL2b and localized in the image by DL2c, the user receives an output with a type of the suspected pathology and a localization of the fetal abnormality as shown in Figure 12. The dashed rectangle enclosed the region comprising the detected pathology. Said dashed rectangle is defined using the bounding box and corresponding to the center position of the box along the x axis, center position of the box along the y axis, box width, box height and possibly box rotation obtained from the object detector DL2c. The output represented in Figure 12 further comprises a message informing the user of a possible "ventricular septal defect".

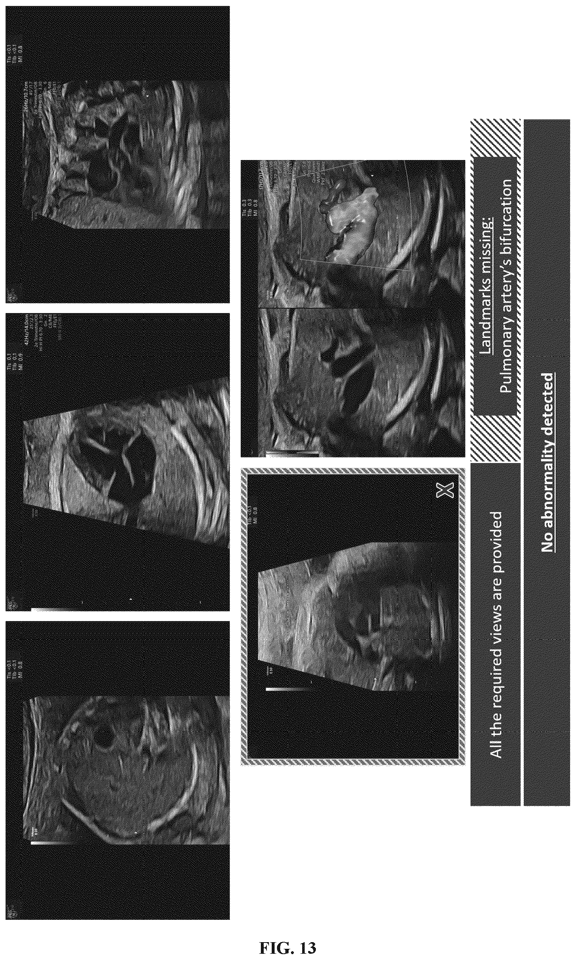

Finally, in the case when ultrasound images were provided manually by user, the first classifier DL1a and the second classifier DL1b treat them in order to identify to which view's category they belong and identify whether the predefined fetal anatomical landmarks associated to the identified view's category are present. If the images provided manually have some fetal anatomical landmarks missing, the user receives a corresponding output such as the one reported in Figure 13. Indeed, in Figure 13, the right ventricular outflow tract (RVOT) view has some missing landmarks (bifurcation of pulmonary artery is not visible). Using this image for fetal examination is therefore associated with a risk of misrecognition of some abnormalities.

The present invention further relates to a computer program product for guiding a user in ultrasound assessment of a fetal organ, the computer program product comprising instructions which, when the program is executed by a computer, cause the computer to automatically carry out the steps of the method according to any one of the embodiments described hereabove.

The computer program product to perform the method as described above may be written as computer programs, code segments, instructions or any combination thereof, for individually or collectively instructing or configuring the processor or computer to operate as a machine or special-purpose computer to perform the operations performed by hardware components. In one example, the computer program product includes machine code that is directly executed by a processor or a computer, such as machine code produced by a compiler. In another example, the computer program product includes higher-level code that is executed by a processor or a computer using an interpreter. Programmers of ordinary skill in the art can readily write the instructions or software based on the block diagrams and the flow charts illustrated in the drawings and the corresponding descriptions in the specification, which disclose algorithms for performing the operations of the method as described above.

The present invention further relates to a computer readable storage medium for guiding a user in ultrasound assessment of a fetal organ comprising instructions which, when the program is executed by a computer, cause the computer to carry out the method according to any one of the embodiments described here above.

According to one embodiment, the computer-readable storage medium is a non-transitory computer-readable storage medium.

Computer programs implementing the method of the present embodiments can commonly be distributed to users on a distribution computer-readable storage medium such as, but not limited to, an SD card, an external storage device, a microchip, a flash memory device, a portable hard drive and software websites. From the distribution medium, the computer programs can be copied to a hard disk or a similar intermediate storage medium. The computer programs can be run by loading the computer instructions either from their distribution medium or their intermediate storage medium into the execution memory of the computer, configuring the computer to act in accordance with the method of this invention. All these operations are well- known to those skilled in the art of computer systems.

The instructions or software to control a processor or computer to implement the hardware components and perform the methods as described above, and any associated data, data files, and data structures, are recorded, stored, or fixed in or on one or more non-transitory computer-readable storage media. Examples of a non-transitory computer-readable storage medium include read-only memory (ROM), random-access memory (RAM), flash memory, CD- ROMs, CD- Rs, CD+ Rs, CD- RWs, CD+ RWs, DVD- ROMs, DVD- Rs, DVD+ Rs, DVD- RWs, DVD+ RWs, DVD- RAMs, BD- ROMs, BD- Rs, BD- R LTHs, BD- Res, magnetic tapes, floppy disks, magneto-optical data storage devices, optical data storage devices, hard disks, solid-state disks, and any device known to one of ordinary skill in the art that is capable of storing the instructions or software and any associated data, data files, and data structures in a non-transitory manner and providing the instructions or software and any associated data, data files, and data structures to a processor or computer so that the processor or computer can execute the instructions. In one example, the instructions or software and any associated data, data files, and data structures are distributed over network-coupled computer systems so that the instructions and software and any associated data, data files, and data structures are stored, accessed, and executed in a distributed fashion by the processor or computer.

While various embodiments have been described and illustrated, the detailed description is not to be construed as being limited hereto. Various modifications can be made to the embodiments by those skilled in the art without departing from the true spirit and scope of the disclosure as defined by the claims.

### EXAMPLES

The present invention is further illustrated by the following examples.

### Example 1:

The image analysis architecture DL1 was dynamically trained on a semi-labeled database with the following views distribution:
- images non belonging to any view (NV) - 33.01%,
- abdominal situs view (V1) - 9.20%,
- four-chamber view (V2) - 23.28%,
- left ventricular outflow tract view (V3) - 10.15%,
- right ventricular outflow tract views (V4) - 11.63%,
- three-vessel views (V5) - 12.73%.

A weighted cost function was implemented in order to take into account this imbalanced database. This database was divided into train and validation parts of 80% and 20% respectively.

On the validation database (the one that was not used for training), for the image analysis structure, the first classifier DL1a algorithm shows a precision of 98.41% and the second classifier DL1b algorithm a precision of 92.91%.

For the diagnostic structure, the first classifier DL2a algorithm shows a precision of 96.72% while the second classifier DL2b algorithm presented a precision of 93.11%.

It is worth mentioning that the obtained results can be improved by enlarging the training database.

### REFERENCES

[Mai2019] Mai, Cara T., et al. "National population-based estimates for major birth defects, 2010-2014." Birth Defects Research 111.18 (2019): 1420-1435.
[Decoufle2001] Decouflé, Pierre, et al. "Increased risk for developmental disabilities in children who have major birth defects: a population-based study." Pediatrics 108.3 (2001): 728-734.
[CDCP2015] Centers for Disease Control and Prevention, National Vital Statistics Report. (2015). Deaths: Final data for 2013. NVSR Volume 64, Number 2. Retrieved from http://www.cdc.gov/nchs/data/nvsr/nvsr64/nvsr64_02.pdf
[Bower2010] Bower, C., Rudy, E., Callaghan, A., Quick, J., & Nassar, N. (2010). Age at diagnosis of birth defects. Birth Defects Research. Part A, Clinical and Molecular Teratology, 88, 251-255.
[TBDR2016] Texas Birth Defects Registry. (2016). Report of defects among 1999-2011 deliveries. Retrieved from http://www.dshs.state.tx.us/birthdefects/data/BD_Data_99-11/Report-of-Birth-Defects-Among-1999-2011-Deliveries.aspx.
[Bensemlali2017] Bensemlali M, Bajolle F, Laux D, et al. Neonatal management and outcomes of prenatally diagnosed CHDs. Cardiol Young. 2017;27(2):344-353. doi:10.1017/S1047951116000639
[Berkley2009] Berkley, Eliza MF, et al. "Utility of fetal echocardiography in postnatal management of infants with prenatally diagnosed congenital heart disease." Prenatal Diagnosis: Published in Affiliation With the International Society for Prenatal Diagnosis 29.7 (2009): 654-658.
[AIUM2003] AIUM. AIUM Practice Guideline for the performance of an antepartum obstetric ultrasound examination. J Ultrasound Med 2003; 22: 1116-1125.
[ACR2003] ACR Practice Guideline for the performance of antepartum obstetrical ultrasound. Am Coll Radiol 2003: 689-695.
[Lee1998] Lee W. Performance of the basic fetal cardiac ultrasound examination. J Ultrasound Med 1998; 17: 601-607.
[Carvalho2013] Carvalho, J. S., et al. "ISUOG Practice Guidelines (updated): sonographic screening examination of the fetal heart." (2013).
[Cdcp2008] CDCP. "Update on overall prevalence of major birth defects--Atlanta, Georgia, 1978-2005." MMWR. Morbidity and mortality weekly report 57.1 (2008) Retrieved from https://www.cdc.gov/mmwr/preview/mmwrhtml/mm5701a2.htm#:~:text=The%20overa ll%20prevalence%20of%20major%20defects%20was%20stable%20from%201978,197 8%20to%2051%2C400%20in%202005.
[Cha2012] Cha, Seulgi, et al. "Recent trends in indications of fetal echocardiography and postnatal outcomes in fetuses diagnosed as congenital heart disease." Korean circulation journal 42.12 (2012): 839-844.
[Clur2012] Clur, S. A., et al. "Prenatal diagnosis of cardiac defects: accuracy and benefit." Prenatal diagnosis 32.5 (2012): 450-455.
[CNEOF2016] Retrieved from http://www.cfef.org/docdoc.php
[Debost2014] Debost-Legrand, Anne, et al. "False positive morphologic diagnoses at the anomaly scan: marginal or real problem, a population-based cohort study." BMC pregnancy and childbirth 14.1 (2014): 112.
[Jouannic2010] Jouannic, J. M. "Anomalies cardiaques foetales: diagnostic prénatal et prise en charge périnatale." EMC (Elsevier Masson SAS), Obstétrique, 5-031-A 38 (2010).
[Lee2013] Lee, Wesley, et al. "AIUM practice guideline for the performance of fetal echocardiography." Journal of Ultrasound in Medicine 32.6 (2013): 1067-1082.
[Mathews2015] Mathews, T. J., et al. "Infant mortality statistics from the 2013 period linked birth/infant death data set." (2015).
[Pinto2013] Pinto, Antonio, et al. "Sources of error in emergency ultrasonography." Critical ultrasound journal 5.S1 (2013): S1.
[Sklansky2016] Sklansky, Mark, and Greggory R. DeVore. "Fetal cardiac screening: what are we (and our guidelines) doing wrong?" Journal of Ultrasound in Medicine 35.4 (2016): 679-681.
[Bensemlali2016] Bensemlali, Myriam, et al. "Discordances between pre-natal and postnatal diagnoses of congenital heart diseases and impact on care strategies." Journal of the American College of Cardiology 68.9 (2016): 921-930.
[vanNisselrooij2020] van Nisselrooij, A. E. L., et al. "Why are congenital heart defects being missed?." Ultrasound in Obstetrics & Gynecology 55.6 (2020): 747-757.

## Claims

1. A system for guiding a user in ultrasound assessment of a fetal organ so as to perform a diagnostic evaluation of the fetal organ development during a medical examination, said ultrasound assessment being based on an ultrasound images sequence comprising multiples predefined required views of the fetal organ, said system comprising:
- input module configured to receive:
o in real time a sequence of 2D ultrasound images, said images comprising multiple predefined required views of the fetal organ, wherein each image comprises at least a portion of the fetal organ;
o a predefined list of view's categories, wherein each view's category is associated to at least one predefined fetal anatomical landmark;
- an image analysis module configured to, for each image:
o provide the image as input to an image analysis structure (DL1) comprising at least one first classifier (DL1a), said first classifier (DL1a) being configured to identify whether the image belongs to any view's category comprised in the predefined list of view's categories and, if so, to identify the view's category to which belongs the image among said predefined list of view's categories;
o provide the image as input to a second classifier (DL1b) of the image analysis structure (DL1), said second classifier (DL1b) being configured to detect the presence of predefined fetal anatomical landmarks in the image;
o add the image to a valid images list whenever:
▪ the first classifier (DL1a) identifies that the image corresponds to one view's category of the predefined list of view's categories; and
▪ the second classifier (DL1b) identifies that a predefined number of fetal anatomical landmarks are present in the image, said fetal anatomical landmarks being associated to said one view's category identified by the first classifier;
o providing the valid images list.

2. The system according to claim **1,** wherein the fetal organ is the fetal heart.

3. The system according to either one of claims **1** or **2,** wherein in the image analysis module the image analysis structure (DL1) comprises a first stage employing a convolutional neural network and wherein the first classifier of the image analysis structure (DL1a) comprises a second stage employing a fully connected neural network receiving as input at least a portion of the output of the first stage of the image analysis structure (DL1).

4. The system according to any one of claims **1** to **3,** whenever at least one image has been provided by the user manually, the image analysis module is further configured to provide the image as input to the first classifier of the image analysis structure (DL1a) and the second classifier of the image analysis structure (DL1a) and whenever the first classifier (DL1a) identifies that the image corresponds to one view's category of the predefined list of view's categories and the second classifier (DL1b) identifies that a predefined number of fetal anatomical landmarks associated to the identified view's category are present in the image, adding said image to a valid images list.

5. The system according to claim **4,** whenever at least one image has been provided by the user manually but is not validated by the second classifier (DL1b), the image analysis module is further configured to provide the image as input to an object detector (DL1c) of the image analysis structure (DL1) comprising a fourth stage configured to receive as input at least a portion of the output of the first stage of the image analysis structure (DL1) and comprising region-based fully convolutional neural network architecture being configured to perform segmentation of the image, so as to classify and localize fetal anatomical landmarks in the image.

6. The system according to any one of claims **1** to **5,** further comprising:
- a diagnostic module that when the valid images list comprises all the predefined required views of the fetal organ is configured to:
o provide a stack of one image of the valid images list as input to a diagnostic structure (DL2), wherein the diagnostic structure (DL2) comprises a first stage employing a convolutional neural network receiving as input the stack of images and providing an output and wherein said diagnostic structure (DL2) comprises a first classifier (DL2a) employing, at a second stage, a fully connected neural network receiving as input at least a portion of the output of the first stage of the diagnostic structure (DL2); the first classifier of the diagnostic structure (DL2a) being configured to discriminate between pathological development and physiological development of the fetal organ;
o whenever the output of the first classifier of the diagnostic structure (DL2a) categorizes the image as comprising a pathological development, providing the image as input to:
• a second classifier (DL2b) of the diagnostic structure (DL2) comprising a third stage employing a fully connected neural network receiving as input at least a portion of the output of the first stage of the diagnostic structure (DL2) and being configured to classify the pathological development into at least one pathology category;
• an object detector (DL2c) of the diagnostic structure (DL2) comprising a fourth stage configured to receive as input at least a portion of the output of the first stage of the diagnostic structure (DL2) and comprising a fully convolutional neural network being configured to perform segmentation of the image and localization of at least one pathological development region in the fetal organ;
- output module configured to:
o output to the user the at least one pathology category obtained from the second classifier (DL2b) and the result of the image segmentation of the image and localization of the pathological development region in the fetal organ obtained from the object detector (DL2c) of the diagnostic structure (DL2);
o output to the user a message to end examination, whenever the output of the first classifier of the diagnostic structure (DL2a) categorizes the image as comprising a physiological development.

7. The system according to claim **6,** wherein the fully convolutional neural network of the fourth stage of the object detector (DL2c) is based on the region-based fully convolutional neural network architecture.

8. The system according to either one of claim **6** or **7,** wherein the first, second classifiers and object detector of a diagnostic structure (DL2) are configured to receive as input a stack of images comprising at least one image.

9. The system according to any one of claims **6** to **8,** wherein the first stage convolutional neural networks of the image analysis structure and of the diagnostic structure have at least one common layer, defined during training.

10. The system according to claim **9,** wherein the image analysis structure and of the diagnostic structure results from a simultaneous training, notably semi-supervised.

11. A computer implemented method for guiding a user in ultrasound assessment of a fetal organ so as to assess the quality of an examination, said method comprising:
- receiving in real time a sequence of 2D ultrasound images, said images comprising multiple predefined required views of the fetal organ, each image comprising at least a portion of the fetal organ (M100);
- receiving a predefined list of view's categories, wherein each view's category is associated to at least one predefined fetal anatomical landmark;
- providing each image as input to an image analysis structure (DL1) comprising at least one first classifier (DL1a), said first classifier (DL1a) being configured to identify whether the image belongs to any view's category comprised in the predefined list of view's categories and, if so, to identify the view's category to which belongs the image among said predefined list of view's categories (M200);
- providing each image as input to a second classifier (DL1b) of the image analysis structure (DL1), said second classifier (DL1b) being configured to detect the presence in the image of predefined fetal anatomical landmarks (M300);
- whenever, for each image:
o the first classifier (DL1a) identifies that the image corresponds to one view's category of the predefined list of view's categories; and
o the second classifier (DL1b) identifies that a predefined number of fetal anatomical landmarks are present in the image, said fetal anatomical landmarks being associated to said one view's category identified by the first classifier (M400),
adding said image to a valid images list (M500);
- providing the valid images list to the user.

12. The method according to claim 11, when the valid images list comprises all the predefined required views of the fetal organ, further comprises providing a message to inform the user that the valid images list comprises all the predefined required views of the fetal organ.

13. The method according to either one of claims 11 or 12, wherein the predefined list of view's categories comprises ultrasound view categories from medical ultrasound guidelines and the predefined fetal anatomical landmarks comprise physiological fetal landmarks from medical guidelines.

14. A computer program product for guiding a user in ultrasound assessment of a fetal organ, the computer program product comprising instructions which, when the program is executed by a computer, cause the computer to automatically carry out the method according to any one of claims **11** to **13.**

15. A computer readable storage medium for guiding a user in ultrasound assessment of a fetal organ comprising instructions which, when the program is executed by a computer, cause the computer to carry out the method according to any one of claims **11** to **13.**

## Patentansprüche

1. System zur Anleitung eines Benutzers bei der Ultraschallbewertung eines fetalen Organs, um eine diagnostische Bewertung der fetalen Organentwicklung während einer medizinischen Untersuchung durchzuführen, wobei die Ultraschallbewertung auf einer Abfolge von Ultraschallbildern basiert, die mehrere vordefinierte erforderliche Ansichten des fetalen Organs umfasst, wobei das System Folgendes umfasst:
- Eingabemodul, eingerichtet zum Empfangen von:
o einer Abfolge von 2D-Ultraschallbildern in Echtzeit, wobei die Bilder mehrere vordefinierte erforderliche Ansichten des fetalen Organs umfassen, wobei jedes Bild mindestens einen Teil des fetalen Organs umfasst;
o einer vordefinierte Liste von Ansichtskategorien, wobei jede Ansichtskategorie mindestens einem vordefinierten fetalen anatomischen Orientierungspunkt zugeordnet ist;
- einem Bildanalysemodul, das für jedes Bild für Folgendes eingerichtet ist:
∘ Bereitstellung des Bildes als Eingabe für eine Bildanalysestruktur (DL1), die mindestens einen ersten Klassifikator (DL1a) umfasst, wobei der erste Klassifikator (DL1a) so eingerichtet ist, dass er identifiziert, ob das Bild zu einer Ansichtskategorie gehört, die in der vordefinierten Liste der Ansichtskategorien enthalten ist, und wenn ja, um die Ansichtskategorie zu identifizieren, zu der das Bild unter den vordefinierten Ansichtskategorien gehört;
∘ Bereitstellung des Bildes als Eingabe für einen zweiten Klassifikator (DL1b) der Bildanalysestruktur (DL1), wobei der zweite Klassifikator (DL1b) so eingerichtet ist, dass er das Vorhandensein vordefinierter fetaler anatomischer Orientierungspunkte im Bild erkennt;
∘ Hinzufügen des Bildes zu einer gültigen Bildliste, wenn:
▪ der erste Klassifikator (DL1a) identifiziert, dass das Bild einer Ansichtskategorie der vordefinierten Liste der Ansichtskategorien entspricht; und
▪ der zweite Klassifikator (DL1b) identifiziert, dass eine vordefinierte Anzahl fetaler anatomischer Orientierungspunkte im Bild vorhanden ist, wobei die fetalen anatomischen Orientierungspunkte der Ansichtskategorie zugeordnet sind, die durch den ersten Klassifikator identifiziert wird;
∘ die gültige Bildliste bereitgestellt wird.

2. System nach Anspruch **1,** wobei das fetale Organ das fetale Herz ist.

3. System nach einem der Ansprüche **1** oder **2,** wobei im Bildanalysemodul die Bildanalysestruktur (DL1) eine erste Stufe umfasst, die ein konvolutionales neurales Netzwerk verwendet, und wobei der erste Klassifikator der Bildanalysestruktur (DL1a) eine zweite Stufe umfasst, die ein vollständig verbundenes neurales Netzwerk verwendet, das mindestens einen Teil der Ausgabe der ersten Stufe der Bildanalysestruktur (DL1) als Eingabe empfängt.

4. System nach einem der Ansprüche **1** bis **3,** wann immer mindestens ein Bild vom Benutzer manuell bereitgestellt wurde, ist das Bildanalysemodul ferner so eingerichtet, dass es das Bild als Eingabe für den ersten Klassifikator der Bildanalysestruktur (DL1a) und den zweiten Klassifikator der Bildanalysestruktur (DL1a) bereitstellt und wann immer der erste Klassifikator (DL1a) identifiziert, dass das Bild der Ansichtskategorie der vordefinierten Liste der Ansichtskategorien entspricht, und der zweite Klassifikator (DL1b) identifiziert, dass eine vordefinierte Anzahl fetaler anatomischer Orientierungspunkte, die der identifizierten Ansichtskategorie zugeordnet sind, im Bild vorhanden sind, wodurch das Bild zu einer gültigen Bildliste hinzugefügt wird.

5. System nach Anspruch **4,** wann immer mindestens ein Bild vom Benutzer manuell bereitgestellt wurde, aber nicht durch den zweiten Klassifikator (DL1b) validiert wird, ist das Bildanalysemodul ferner so eingerichtet, dass es das Bild als Eingabe für einen Objektdetektor (DL1c) der Bildanalysestruktur (DL1) bereitstellt, die eine vierte Stufe umfasst, die so eingerichtet ist, dass sie als Eingabe mindestens einen Teil der Ausgabe der ersten Stufe der Bildanalysestruktur (DL1) empfängt und umfassend eine regionsbasierte, vollständig konvolutionale neuronale Netzwerkarchitektur, die so eingerichtet ist, dass sie eine Segmentierung des Bildes durchführt, um fetale anatomische Orientierungspunkte im Bild zu klassifizieren und zu lokalisieren.

6. System nach einem der Ansprüche 1 bis 5, ferner umfassend:
- ein Diagnosemodul, dass, wenn die Liste der gültigen Bilder alle vordefinierten erforderlichen Ansichten des fetalen Organs umfasst, für Folgendes eingerichtet ist:
o Bereitstellung eines Stapels eines Bildes der gültigen Bildliste als Eingabe für eine Diagnosestruktur (DL2), wobei die Diagnosestruktur (DL2) eine erste Stufe umfasst, die ein konvolutionales neurales Netzwerk verwendet, das den Stapel von Bildern als Eingabe empfängt und eine Ausgabe bereitstellt, und wobei die Diagnosestruktur (DL2) einen ersten Klassifikator (DL2a) umfasst, der in einer zweiten Stufe verwendet, ein vollständig verbundenes neuronales Netzwerk, das mindestens einen Teil der Ausgabe der ersten Stufe der Diagnosestruktur (DL2) als Eingabe empfängt; wobei der erste Klassifikator der Diagnosestruktur (DL2a) so eingerichtet ist, dass er zwischen pathologischer Entwicklung und physiologischer Entwicklung des fetalen Organs unterscheidet;
∘ wenn die Ausgabe des ersten Klassifikators der Diagnosestruktur (DL2a) das Bild als eine pathologische Entwicklung kategorisiert und das Bild als Eingabe für Folgendes bereitstellt:
• einen zweiten Klassifikator (DL2b) der Diagnosestruktur (DL2), der eine dritte Stufe umfasst, die ein vollständig verbundenes neuronales Netzwerk verwendet, das mindestens einen Teil der Ausgabe der ersten Stufe der Diagnosetruktur (DL2) als Eingabe empfängt und so eingerichtet ist, dass es die pathologische Entwicklung in mindestens eine Pathologiekategorie einstuft;
• einen Objektdetektor (DL2c) der Diagnosestruktur (DL2), der eine vierte Stufe umfasst, die so eingerichtet ist, dass sie mindestens einen Teil der Ausgabe der ersten Stufe der Diagnosestruktur (DL2) als Eingabe empfängt, und der ein vollständig konvolutionales neurales Netzwerk umfasst, das so eingerichtet ist, dass es die Segmentierung des Bildes und die Lokalisierung mindestens eines pathologischen Entwicklungsbereichs im fetalen Organ durchführt;
- Ausgabemodul, eingerichtet für:
∘ Ausgabe an den Benutzer der mindestens einen Pathologiekategorie, die aus dem zweiten Klassifikator (DL2b) gewonnen wurde, und des Ergebnisses der Bildsegmentierung des Bildes und der Lokalisierung des pathologischen Entwicklungsbereichs im fetalen Organ, die aus dem Objektdetektor (DL2c) der Diagnosestruktur (DL2) gewonnen wurde, an den Benutzer;
∘ Ausgabe einer Meldung an den Benutzer, um die Untersuchung zu beenden, wenn die Ausgabe des ersten Klassifikators der Diagnosestruktur (DL2a) das Bild als eine physiologische Entwicklung kategorisiert.

7. System nach Anspruch **6,** wobei das vollkonvolutionale neuronale Netzwerk der vierten Stufe des Obj ektdetektors (DL2c) auf der regionsbasierten vollkonvolutionalen neuronalen Netzwerkarchitektur basiert.

8. System nach einem der Ansprüche **6** oder **7,** wobei die ersten, zweiten Klassifikatoren und der Objektdetektor einer Diagnosestruktur (DL2) so eingerichtet sind, dass sie als Eingabe einen Stapel von Bildern empfangen, der mindestens ein Bild umfasst.

9. System nach einem der Ansprüche **6** bis **8,** wobei die konvolutionalen neuronalen Netze der ersten Stufe der Bildanalysestruktur und der Diagnosestruktur mindestens eine gemeinsame Schicht aufweisen, die während des Trainings definiert ist.

10. System nach Anspruch **9,** wobei sich die Bildanalysestruktur und die Diagnosestruktur aus einem gleichzeitigen Training, insbesondere semi-beaufsichtigt, ergibt.

11. Computerimplementiertes Verfahren zur Anleitung eines Benutzers bei der Ultraschallbewertung eines fetalen Organs, um die Qualität einer Untersuchung zu beurteilen, wobei das Verfahren Folgendes umfasst:
- Empfangen einer Abfolge von 2D-Ultraschallbildern in Echtzeit, wobei die Bilder mehrere vordefinierte erforderliche Ansichten des fetalen Organs umfassen, wobei jedes Bild mindestens einen Teil des fetalen Organs (M100) umfasst;
- Empfangen einer vordefinierten Liste von Ansichtskategorien, wobei jede Ansichtskategorie mindestens einem vordefinierten fetalen anatomischen Orientierungspunkt zugeordnet ist;
- Bereitstellen jedes Bildes als Eingabe für eine Bildanalysestruktur (DL1), die mindestens einen ersten Klassifikator (DL1a) umfasst, wobei der erste Klassifikator (DL1a) so eingerichtet ist, dass er identifiziert, ob das Bild zu einer Ansichtskategorie gehört, die in der vordefinierten Liste der Ansichtskategorien enthalten ist, und wenn ja, um die Ansichtskategorie zu identifizieren, zu der das Bild unter der vordefinierten Liste der Ansichtskategorien (M200) gehört;
- Bereitstellen jedes Bildes als Eingabe für einen zweiten Klassifikator (DL1b) der Bildanalysestruktur (DL1), wobei der zweite Klassifikator (DL1b) so eingerichtet ist, dass er das Vorhandensein von vordefinierten fetalen anatomischen Orientierungspunkten (M300) im Bild erkennt;
- wenn, für jedes Bild:
∘ der erste Klassifikator (DL1a) identifiziert, dass das Bild der Ansichtskategorie der vordefinierten Liste der Ansichtskategorien entspricht; und
∘ der zweite Klassifikator (DL1b) identifiziert, dass eine vordefinierte Anzahl fetaler anatomischer Orientierungspunkte im Bild vorhanden ist, wobei die fetalen anatomischen Orientierungspunkte der Ansichtskategorie zugeordnet sind, die durch den ersten Klassifikator (M400) identifiziert wird,
Hinzufügen des Bildes zu einer gültigen Bildliste (M500);
- Bereitstellen der gültigen Bildliste an den Benutzer.

12. Das Verfahren nach Anspruch 11, wenn die Liste der gültigen Bilder alle vordefinierten erforderlichen Ansichten des fetalen Organs umfasst, umfasst ferner das Bereitstellen einer Nachricht, um den Benutzer darüber zu informieren, dass die Liste der gültigen Bilder alle vordefinierten erforderlichen Ansichten des fetalen Organs umfasst.

13. Verfahren nach einem der Ansprüche 11 oder 12, wobei die vordefinierte Liste der Ansichtskategorien Ultraschallansichtskategorien aus medizinischen Ultraschallrichtlinien umfasst und die vordefinierten fetalen anatomischen Orientierungspunkte physiologische fetale Orientierungspunkte aus medizinischen Richtlinien umfassen.

14. Computerprogrammprodukt zur Anleitung eines Benutzers bei der Ultraschallbewertung eines fetalen Organs, wobei das Computerprogrammprodukt Anweisungen umfasst, die, wenn das Programm von einem Computer ausgeführt wird, den Computer dazu veranlassen, das Verfahren nach einem der Ansprüche **11** bis **13** automatisch auszuführen.

15. Computerlesbares Speichermedium zur Anleitung eines Benutzers bei der Ultraschallbewertung eines fetalen Organs, umfassend Anweisungen, die, wenn das Programm von einem Computer ausgeführt wird, den Computer veranlassen, das Verfahren nach einem der Ansprüche **11** bis **13** auszuführen.

## Revendications

1. Un système pour guider un utilisateur dans l'évaluation par ultrasons d'un organe fœtal afin de réaliser une évaluation diagnostique du développement de l'organe fœtal lors d'un examen médical, ladite évaluation par ultrasons étant basée sur une séquence d'images ultrasonores comprenant plusieurs vues prédéfinies requises de l'organe fœtal, ledit système comprenant :
- un module d'entrée configuré pour recevoir :
∘ en temps réel une séquence d'images ultrasonores 2D, lesdites images comprenant plusieurs vues prédéfinies requises de l'organe fœtal, dans lequel chaque image comprenant au moins une partie de l'organe fœtal ;
∘ une liste prédéfinie de catégories de vues, dans lequel chaque catégorie de vues est associée à au moins un repère anatomique fœtal prédéfini,
- un module d'analyse d'images configuré pour, pour chaque image :
∘ fournir l'image en entrée d'une structure d'analyse d'image (DL1) comprenant au moins un premier classificateur (DL1a), ledit premier classificateur (DL1a) étant configuré pour identifier si l'image appartient à l'une des catégories de vues comprises dans la liste prédéfinie de catégories de vues et, le cas échéant, pour identifier la catégorie de vues à laquelle l'image appartient parmi ladite liste prédéfinie de catégories de vues ;
- fournir l'image en entrée d'un second classificateur (DL1b) de la structure d'analyse d'image (DL1), ledit second classificateur (DL1b) étant configuré pour détecter la présence dans l'image de repères anatomiques fœtaux prédéfinis ;
- ajouter l'image à une liste d'images valides dès lors :
∘ que le premier classificateur (DL1a) identifie que l'image correspond à une catégorie de vues de la liste prédéfinie de catégories de vues ; et
∘ que le second classificateur (DL1b) identifie qu'un nombre prédéfini de repères anatomiques fœtaux sont présents dans l'image, les repères anatomiques fœtaux étant associés à ladite catégorie de vues identifiée par le premier classificateur ;
- fournir la liste d'images valides.

2. Le système selon la revendication **1,** dans lequel l'organe fœtal est le cœur fœtal.

3. Le système selon l'une quelconque des revendications **1** ou **2,** dans lequel, dans le module d'analyse d'images, la structure d'analyse d'images (DL1) comprend un premier étage utilisant un réseau de neurones convolutif et dans lequel le premier classificateur de la structure d'analyse d'images (DL1a) comprend un second étage utilisant un réseau de neurones entièrement connecté recevant en entrée au moins une partie de la sortie du premier étage de la structure d'analyse d'images (DL1).

4. Le système selon la revendication **3,** dans lequel, lorsqu'au moins une image a été fournie manuellement par l'utilisateur, le module d'analyse d'images est en outre configuré pour fournir ladite image en entrée au premier classificateur de la structure d'analyse d'images (DL1a) et au second classificateur de la structure d'analyse d'images (DL1b), et dès lors que le premier classificateur (DL1a) identifie que l'image correspond à une catégorie de vues de la liste prédéfinie de catégories de vues et que le second classificateur (DL1b) identifie qu'un nombre prédéfini de repères anatomiques fœtaux associés à la catégorie de vues identifiée sont présents dans l'image, ajouter ladite image à une liste d'images valides.

5. Le système selon la revendication **4,** dans lequel, lorsqu'au moins une image a été fournie manuellement par l'utilisateur mais n'est pas validée par le second classificateur (DL1b), le module d'analyse d'images est en outre configuré pour fournir ladite image en entrée à un détecteur d'objets (DL1c) de la structure d'analyse d'images (DL1), ledit détecteur comprenant un quatrième étage configuré pour recevoir en entrée au moins une partie de la sortie du premier étage de la structure d'analyse d'images (DL1), et comprenant une architecture de réseau de neurones entièrement convolutif, basé sur les régions, configurée pour faire une segmentation des images, afin de classifier et localiser les repères anatomiques fœtaux de l'image.

6. Le système selon l'une quelconque des revendications **1** à **5,** comprenant en outre :
- un module de diagnostic qui, lorsque la liste d'images valides comprend toutes les vues prédéfinies requises de l'organe fœtal, est configuré pour :
∘ fournir une pile d'au moins une image de la liste d'images valides en entrée d'une structure de diagnostic (DL2), ladite structure de diagnostic (DL2) comprenant un premier étage utilisant un réseau de neurones convolutif recevant en entrée la pile d'images et produisant une sortie, dans lequel ladite structure de diagnostic (DL2) comprend un premier classificateur (DL2a) utilisant, à une second étage, un réseau de neurones complètement connecté recevant en entrée au moins une partie de la sortie du premier étage de la structure de diagnostic (DL2) ; le premier classificateur de la structure de diagnostic (DL2a) étant configuré pour discriminer entre un développement pathologique et un développement physiologique de l'organe fœtal ;
∘ lorsque la sortie du premier classificateur de la structure de diagnostic (DL2a) classe l'image comme présentant un développement pathologique, fournir l'image en entrée à :
▪ un second classificateur (DL2b) de la structure de diagnostic (DL2) comprenant un troisième étage utilisant un réseau de neurones complètement connecté recevant en entrée au moins une partie de la sortie du premier étage de la structure de diagnostic (DL2) et étant configuré pour classifier le développement pathologique en au moins une catégorie de pathologie ;
▪ un détecteur d'objets (DL2c) de la structure de diagnostic (DL2) comprenant une quatrième étape configurée pour recevoir en entrée au moins une partie de la sortie du premier étage de la structure de diagnostic (DL2) et comprenant un réseau de neurones convolutif configuré pour effectuer une segmentation de l'image et une localisation d'au moins une région de développement pathologique dans l'organe fœtal ;
- un module de sortie configuré pour :
∘ fournir à l'utilisateur au moins une catégorie de pathologie obtenue via le second classificateur (DL2b) et le résultat de la segmentation de l'image ainsi que la localisation de la région de développement pathologique dans l'organe fœtal obtenue via le détecteur d'objets (DL2c) de la structure de diagnostic (DL2) ;
∘ afficher à l'utilisateur un message indiquant la fin de l'examen lorsque la sortie du premier classificateur de la structure de diagnostic (DL2a) classe l'image comme présentant un développement physiologique.

7. Le système selon la revendication **6,** dans lequel le réseau de neurones entièrement convolutif du quatrième étage du détecteur d'objets (DL2c) est basé sur une architecture de réseau de neurones entièrement convolutif basé sur les régions.

8. Le système selon l'une quelconque des revendications **6** ou **7,** dans lequel le premier classificateur, le second classificateur et le détecteur d'objets de la structure de diagnostic (DL2) sont configurés pour recevoir en entrée une pile d'images comprenant au moins une image.

9. Le système selon l'une quelconque des revendications **6** à **8,** dans lequel les réseaux de neurones convolutif du premier étage de la structure d'analyse d'images et de la structure de diagnostic comprennent au moins une couche commune, définie lors de l'entraînement.

10. Le système selon la revendication **9,** dans lequel la structure d'analyse d'images et la structure de diagnostic résultent d'un entraînement simultané, notamment semisupervisé.

11. Une méthode mise en œuvre par ordinateur permettant de guider un utilisateur dans l'évaluation par ultrasons d'un organe fœtal afin d'évaluer la qualité d'un examen, ladite méthode comprenant :
- la réception en temps réel d'une séquence d'images ultrasonores 2D, lesdites images comprenant plusieurs vues prédéfinies requises de l'organe fœtal, chaque image comprenant au moins une partie de l'organe fœtal (M100) ;
- la réception d'une liste prédéfinie de catégories de vues, chaque catégorie de vues étant associée à au moins un repère anatomique fœtal prédéfini ;
- la fourniture de chaque image en entrée d'une structure d'analyse d'images (DL1) comprenant au moins un premier classificateur (DL1a), ledit premier classificateur (DL1a) étant configuré pour identifier si l'image appartient à une catégorie de vues comprise dans la liste prédéfinie de catégories de vues et, le cas échéant, pour identifier la catégorie de vues à laquelle appartient l'image parmi ladite liste prédéfinie de catégories de vues ;
- la fourniture de chaque image en entrée d'un second classificateur (DL1b) de la structure d'analyse d'images (DL1), ledit second classificateur (DL1b) étant configuré pour détecter la présence dans l'image de repères anatomiques fœtaux prédéfinis (M300) ;
- lorsque, pour chaque image :
∘ le premier classificateur (DL1a) identifie que l'image correspond à une catégorie de vues de la liste prédéfinie de catégories de vues ; et
∘ le second classificateur (DL1b) identifie qu'un nombre prédéfini de repères anatomiques fœtaux associés à ladite catégorie de vues identifiée par le premier classificateur sont présents dans l'image (M400),
- ajouter ladite image à une liste d'images valides (M500) ;
- fournir la liste d'images valides à l'utilisateur.

12. La méthode selon la revendication **11,** dans laquelle, lorsque la liste d'images valides comprend toutes les vues prédéfinies requises de l'organe fœtal, la méthode comprend en outre l'affichage d'un message informant l'utilisateur que la liste d'images valides est complète.

13. La méthode selon l'une quelconque des revendications **11** ou **12,** dans laquelle la liste prédéfinie de catégories de vues comprend des catégories de vues ultrasonores provenant de directives médicales et les repères anatomiques fœtaux prédéfinis comprennent des repères physiologiques fœtaux issus de directives médicales.

14. Un produit programme d'ordinateur permettant de guider un utilisateur dans l'évaluation par ultrasons d'un organe fœtal, ledit produit programme d'ordinateur comprenant des instructions qui, lorsqu'elles sont exécutées par un ordinateur, amènent l'ordinateur à exécuter la méthode selon l'une quelconque des revendications **11** à **13.**

15. Un support de stockage informatique lisible par ordinateur permettant de guider un utilisateur dans l'évaluation par ultrasons d'un organe fœtal, comprenant des instructions qui, lorsque le programme est exécuté par un ordinateur, amènent l'ordinateur à exécuter la méthode selon l'une quelconque des revendications **11** à **13.**
